# EUROPEAN PATENT SPECIFICATION

(11) **EP 4 071 475 B1**
(45) Date of publication and mention of the grant of the patent: **01.11.2023**
(21) Application number: 22167499.7
(22) Date of filing: 08.04.2022
(51) Int. Cl.: G01N 33/543

(54) **ASSAY DEVICE**
TESTVORRICHTUNG
DISPOSITIF D'ANALYSE

(30) Priority: 08.04.2021 WO PCT/CN2021/085947
(43) Date of publication of application: 12.10.2022
(73) Proprietor: Abbott Rapid Diagnostics International Unlimited Company, Dublin 2 (IE)
(72) Inventor: CHEN, Jet, 2 Dublin (IE); GOU, Larkin, 2 Dublin (IE); WANG, Jianfeng, 2 Dublin (IE); GAO, Amasong, 2 Dublin (IE); HU, Hyde, 2 Dublin (IE)
(74) Representative: Redhouse, Juliet Lauren

(56) References cited:
- WO-A2-99/46591
- WO-A2-2006/080021
- US-A1- 2020 345 286

## Description

### FIELD

Provided herein is technology relating to a lateral flow assay cassette and related methods, kits, systems, and uses to provide an assay for detecting pathogen antigens and/or antibodies specific for pathogen antigens in patient samples.

### BACKGROUND

Human pathogens (e.g., microbes such as viruses, prokaryotes (e.g., bacteria), and eukaryotes (e.g., fungi and protozoan parasites)) cause human disease and hundreds of millions of deaths worldwide. While treatments exist to prevent suffering and death from many human pathogens, their effectiveness often depends on timely diagnosis to identify the etiological agent and determine a proper course of treatment. Accordingly, rapid testing devices for identifying disease-causing agents are needed.

### SUMMARY

Aspects of the invention are as set out in the appended claims.

Lateral flow assays provide technologies for qualitatively detecting and/or quantitatively measuring analytes in a short time using antigen-antibody interaction (e.g., using immunochromatography). These tests typically use an assay device in the form of a lateral flow assay test strip or a device in which the lateral flow assay test strip is mounted inside a plastic case. See, e.g., Int'l Pat. App. Pub. No. WO2011102563A1: U.S. Pat. No. 8,828,739. Assay devices comprising lateral flow assay test strips provide a rapid, point-of-care assay for detecting human pathogens (e.g., antigens from human pathogens and/or human antibodies produced against antigens from human pathogens). The technology provided herein relates to improvements to assay devices. In particular, the technology provided herein relates to an assay device (e.g., a test cassette comprising a case (e.g., a plastic case) and a lateral flow assay test strip) to detect an infection in a patient (e.g., a pathogenic (e.g., viral, bacterial, fungal, parasitic) infection in a patient), e.g., to detect pathogenic (e.g., viral, bacterial, fungal, parasitic) antigens and/or to detect antibodies specific for pathogenic (e.g., viral, bacterial, fungal, parasitic) antigens in a sample from a patient.

For example, in some embodiments, the technology provides an assay device comprising a lateral flow assay test strip as described herein and a capillary tube component configured to provide a metered sample to the lateral flow assay test strip. In some embodiments, the assay device comprises a depressible air chamber in communication with a sample receiving area (e.g., a sample collection port) and the capillary tube component. In some embodiments, the assay device comprises a capillary tube connecting the sample receiving area (e.g., sample collection port) to a buffer well and the depressible air chamber provides a metered sample to the buffer well.

In some embodiments, the technology provides an assay device as described herein, a lancet or other device to prick a finger and provide a blood sample, and a sample transfer device such as a capillary tube, specimen dropper, or pipette. In some embodiments, the technology provides a kit comprising an assay device as described herein, a lancet or other device to prick a finger and provide a blood sample, and a sample transfer device such as a capillary tube, specimen dropper, or pipette.

In some embodiments, the technology provides a self-test assay device (e.g., for detecting anti-pathogen IgG antibodies) and a capillary component configured to provide a metered sample. In some embodiments, the self-test assay device comprises a depressible air chamber in communication with a sample receiving area. In some embodiments, the self-test assay device comprises a capillary channel connecting the sample receiving area to a buffer well.

Accordingly, in some embodiments, the technology relates to an assay device for detecting human IgG antibodies specific for a pathogen and/or human IgM antibodies specific for a pathogen. For example, in some embodiments, the technology provides an assay device comprising a recombinant antigen from a pathogen; an anti-human IgG antibody; and an anti-human IgM antibody (e.g., in some embodiments, the assay device comprises a lateral flow assay test strip comprising a recombinant antigen from a pathogen; an anti-human IgG antibody; and an anti-human IgM antibody). In some embodiments, the anti-human IgG antibody is a monoclonal antibody. In some embodiments, the anti-human IgM antibody is a monoclonal antibody. In some embodiments, the recombinant antigen comprises a label. In some embodiments, the recombinant antigen comprises a gold colloid label. In some embodiments, the recombinant antigen comprises a latex bead label. In some embodiments, the assay device comprises a first test line comprising the anti-human IgG antibody and comprising a second test line comprising the anti-human IgM antibody. In some embodiments, a first lateral flow assay test strip comprises the first test line and a second lateral flow assay test strip comprises the second test line. In some embodiments, one lateral flow assay test strip comprises the first test line and the second test line. In some embodiments, the anti-human IgG antibody is a mouse antibody. In some embodiments, the anti-human IgM antibody is a mouse antibody. In some embodiments, the assay device further comprises a control (e.g., in some embodiments, the assay device comprises a lateral flow assay test strip comprising a control). In some embodiments, the control is a rabbit IgG comprising a label. In some embodiments, the assay device comprises a control line comprising an anti-rabbit antibody (e.g., in some embodiments, the assay device comprises a lateral flow assay test strip comprising an anti-rabbit antibody). In some embodiments, the anti-rabbit antibody is an IgG. In some embodiments, the anti-rabbit antibody is a goat antibody. In some embodiments, the assay device comprises a label pad, wherein the label pad comprises a recombinant antigen from a pathogen (e.g., in some embodiments, the assay device comprises a lateral flow assay test strip comprising a label pad, wherein the label pad comprises a recombinant antigen from a pathogen).

In some embodiments, the technology provides an assay device for detecting a pathogen. For example, in some embodiments, the technology provides an assay device comprising a first anti-pathogen antibody and a second anti-pathogen antibody (e.g., in some embodiments, the assay device comprises a lateral flow assay test strip comprising a first anti-pathogen antibody and a second anti-pathogen antibody). In some embodiments, the first anti-pathogen antibody is immobilized (e.g., in some embodiments, the first anti-pathogen antibody is immobilized on a lateral flow assay test strip). In some embodiments, the second anti-pathogen antibody comprises a label. In some embodiments, the first anti-pathogen antibody and the second anti-pathogen antibody recognize different epitopes of the pathogen. In some embodiments, the assay device comprises a sample pad and the sample pad comprises the second anti-pathogen antibody (e.g., in some embodiments, the assay device comprises a lateral flow assay test strip comprising a sample pad and the sample pad comprises the second anti-pathogen antibody). In some embodiments, the lateral flow device comprises a test line and the test line comprises the first anti-pathogen antibody (e.g., in some embodiments, the assay device comprises a lateral flow assay test strip comprising a test line and the test line comprises the first anti-pathogen antibody).

The technology described herein also provides embodiments of methods for detecting IgG antibodies and/or IgM antibodies specific for a pathogen. For example, in some embodiments, the technology provides a method comprising providing an assay device (e.g., an assay device comprising a lateral flow assay test strip) comprising a recombinant antigen from a pathogen, an anti-human IgG antibody, and an anti-human IgM antibody; contacting a blood sample to the assay device; and observing a detectable signal at a first test line indicating the presence of IgG antibodies specific for the pathogen in the sample and/or observing a detectable signal at a second test line indicating the presence of IgM antibodies specific for the pathogen in the sample. In some embodiments, the assay device comprises a lateral flow assay test strip comprising the recombinant antigen from a pathogen, the anti-human IgG antibody, and the anti-human IgM antibody. In some embodiments, methods comprise contacting a blood sample to the lateral flow assay test strip; and observing a detectable signal at a first test line of the lateral flow assay test strip indicating the presence of IgG antibodies specific for the pathogen in the sample and/or observing a detectable signal at a second test line of the lateral flow assay test strip indicating the presence of IgM antibodies specific for the pathogen in the sample.

In some embodiments, a first lateral flow assay test strip comprises the first test line and a second lateral flow assay test strip comprises the second test line. In some embodiments, one lateral flow assay test strip comprises the first test line and the second test line. In some embodiments, the anti-human IgG antibody is a monoclonal antibody. In some embodiments, the anti-human IgM antibody is a monoclonal antibody. In some embodiments, the recombinant antigen comprises a label. In some embodiments, the recombinant antigen comprises a gold colloid label. In some embodiments, the recombinant antigen comprises a latex bead label.

In some embodiments, the assay device comprises a first test line comprising the anti-human IgG antibody and the assay device comprises a second test line comprising the anti-human IgM antibody (e.g., in some embodiments, the assay device comprises a lateral flow assay test strip comprising the anti-human IgG antibody and the assay device comprises a lateral flow assay test strip comprising a second test line comprising the anti-human IgM antibody). In some embodiments, a first lateral flow test strip comprises the first test line and a second lateral flow test strip comprises the second test line. In some embodiments, one lateral flow test strip comprises the first test line and the second test line. In some embodiments, the anti-human IgG antibody is a mouse antibody. In some embodiments, the anti-human IgM antibody is a mouse antibody.

In some embodiments, the technology provides methods for detecting a pathogen. For example, in some embodiments, methods comprise providing an assay device (e.g., comprising a lateral flow assay test strip) comprising a first anti-pathogen antibody and a second anti-pathogen antibody; contacting a blood sample to the lateral flow device; and observing a detectable signal at a test line indicating the presence of the pathogen in the sample. In some embodiments, the lateral flow assay test strip comprises the first anti-pathogen antibody and the second anti-pathogen antibody and methods comprise contacting a blood sample to the lateral flow assay test strip and observing a detectable signal at a test line of the lateral flow assay test strip indicating the presence of the pathogen in the sample. In some embodiments, the first anti-pathogen antibody is immobilized. In some embodiments, the second anti-pathogen antibody comprises a label. In some embodiments, the first anti-pathogen antibody and the second anti-pathogen antibody recognize different epitopes of the pathogen. In some embodiments, a sample pad (e.g., a sample pad of the lateral flow assay test strip) comprises the second anti-pathogen antibody. In some embodiments, a test line (e.g., a test line of the lateral flow assay test strip) comprises the first anti-pathogen antibody.

The technology finds use in detecting a human IgG antibody specific for a pathogen. In some embodiments, the technology relates to use of assay device as described herein (e.g., comprising a lateral flow assay test strip) to detect a human IgG antibody specific for a pathogen. The technology finds use in detecting a human IgM antibody specific for a pathogen. In some embodiments, the technology relates to use of an assay device as described herein (e.g., comprising a lateral flow assay test strip) to detect a human IgM antibody specific for a pathogen.

In some embodiments, the technology provides an assay device (e.g., an assay device comprising a lateral flow assay test strip) comprising a recombinant pathogen antigen and an anti-human IgG antibody. In some embodiments, the anti-human IgG antibody is a monoclonal antibody. In some embodiments, the recombinant pathogen antigen comprises a label. In some embodiments, the recombinant pathogen antigen comprises a gold colloid label. In some embodiments, the recombinant pathogen antigen comprises a latex bead label. In some embodiments, the assay device (e.g., an assay device comprising a lateral flow assay test strip) comprises a test line comprising the anti-human IgG antibody. In some embodiments, the anti-human IgG antibody is a mouse antibody. In some embodiments, the assay device (e.g., an assay device comprising a lateral flow assay test strip) further comprises a control. In some embodiments, the control is a rabbit IgG comprising a label. In some embodiments, the assay device (e.g., an assay device comprising a lateral flow assay test strip) further comprises a control line comprising an anti-rabbit antibody. In some embodiments, the anti-rabbit antibody is an IgG. In some embodiments, the anti-rabbit antibody is a goat antibody. In some embodiments, the assay device comprises a label pad and the label pad comprises the recombinant pathogen antigen.

In some embodiments, the assay device comprises a lateral flow assay test strip comprising a recombinant pathogen antigen and an anti-human IgG antibody. In some embodiments, the anti-human IgG antibody is a monoclonal antibody. In some embodiments, the recombinant pathogen antigen comprises a label. In some embodiments, the recombinant pathogen antigen comprises a gold colloid label. In some embodiments, the recombinant pathogen antigen comprises a latex bead label. In some embodiments, the lateral flow assay test strip comprises a test line comprising the anti-human IgG antibody. In some embodiments, the anti-human IgG antibody is a mouse antibody. In some embodiments, the lateral flow assay test strip further comprises a control. In some embodiments, the control is a rabbit IgG comprising a label. In some embodiments, the lateral flow assay test strip further comprises a control line comprising an anti-rabbit antibody. In some embodiments, the anti-rabbit antibody is an IgG. In some embodiments, the anti-rabbit antibody is a goat antibody. In some embodiments, the lateral flow assay test strip comprises a label pad and the label pad comprises the recombinant pathogen antigen.

In some embodiments, the technology provides a method for detecting IgG antibodies specific for a pathogen. For example, in some embodiments, methods comprise providing an assay device comprising a recombinant pathogen antigen and an anti-human IgG antibody; contacting a blood sample to the assay device; and observing a detectable signal at a test line indicating the presence of IgG antibodies specific for the pathogen in the sample. In some embodiments, the anti-human IgG antibody is a monoclonal antibody. In some embodiments, the recombinant pathogen antigen comprises a label. In some embodiments, the recombinant pathogen antigen comprises a gold colloid label. In some embodiments, the recombinant pathogen antigen comprises a latex bead label. In some embodiments, the assay device comprises a test line comprising the anti-human IgG antibody. In some embodiments, the anti-human IgG antibody is a mouse antibody.

In some embodiments, the technology provides use of an assay device as described herein to detect a human IgG antibody specific for a pathogen. In some embodiments, the technology provides use of an assay device as described herein (e.g., for detecting anti-pathogen antibodies) to detect the pathogen.

For example, in some embodiments, the technology provides an assay device comprising a lateral flow assay test strip; a capillary tube comprising a large end, a center portion, and a small end; a sample collection port in fluid communication with the small end of the capillary tube; a buffer well in fluid communication with the large end of the capillary tube and with the lateral flow assay test strip; a depressible chamber in fluid communication with the center portion of the capillary tube, wherein the depressible chamber is adapted to move a sample in the capillary tube into the buffer well. In some embodiments, the assay device further comprises a housing comprising a signal window through which is visible at least a portion of the capillary tube; a transfer button coupled to the compressible chamber; a buffer well in fluid communication with the buffer well; and a test results viewing window s through which is visible at least a portion of the lateral flow test strip. In some embodiments, depressing the depressible chamber moves a metered sample in the capillary tube into the buffer well. In some embodiments, the depressible chamber in an expanded state comprises air and the depressible chamber in a depressed state displaces air into the capillary tube to move the sample to the buffer well. In some embodiments, providing a buffer into the buffer well provides a buffer in the buffer well, said buffer in the buffer well mixes with the sample to provide a buffered sample, and said buffered sample contacts the lateral flow assay test strip to initiate a lateral flow assay. In some embodiments, the lateral flow assay test strip comprises a labeled recombinant antigen; and an anti-human antibody. In some embodiments, the anti-human antibody is immobilized on the lateral flow test strip. In some embodiments, the lateral flow assay test strip comprises a first antibody and a second antibody. In some embodiments, the first antibody is immobilized on the lateral flow test strip and the second antibody comprises a label. Additional embodiments will be apparent to persons skilled in the relevant art based on the teachings contained herein.

### BRIEF DESCRIPTION OF THE DRAWINGS

These and other features, aspects, and advantages of the present technology will become better understood with regard to the following drawings.

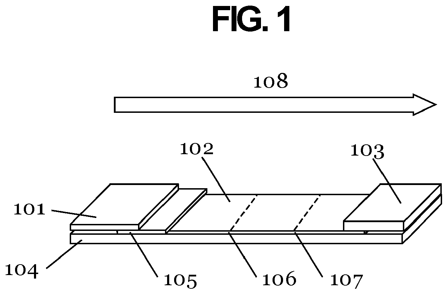

FIG. 1 is a schematic drawing of a device for detecting IgG and/or IgM antibodies against a pathogen in a sample. 101: sample pad; 102: nitrocellulose membrane; 103: absorbent pad; 104: plastic backing; 105: label pad; 106: test line; 107: control line. The direction of flow is indicated by arrow 108.

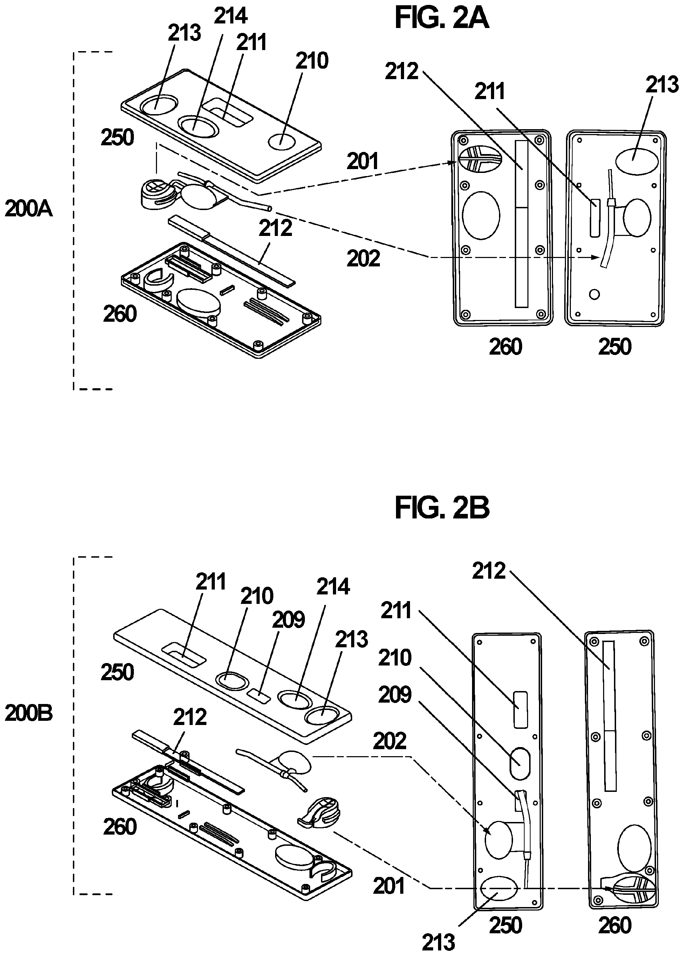

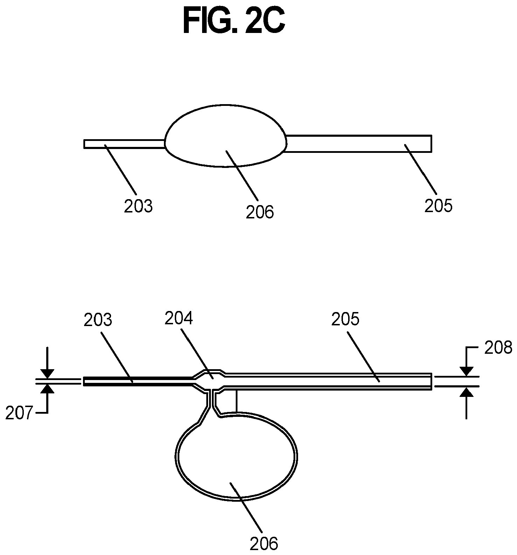

FIG. 2A is a schematic drawing of an embodiment 200A of a self-test device as described herein. 250: top panel; 260: bottom panel; 201: pedestal; 202: blood transfer part comprising a blood sample capillary tube and compressible chamber; 210: buffer well; 211: test results viewing window; 213: sample collection port (e.g., comprising the pedestal 201); 214: window for a user of the self-test device to access (e.g., contact and compress) a compressible chamber (see FIG. 2C, 206).
FIG. 2B is a schematic drawing of an embodiment 200B of a self-test device as described herein. 250: top panel; 260: bottom panel; 201: pedestal; 202: blood transfer part comprising a blood sample capillary tube and compressible chamber; 209: signal window; 210: buffer well; 211: test results viewing window; 213: sample collection port (e.g., comprising the pedestal 201); 214: window for a user of the self-test device to access (e.g., contact and compress) a compressible chamber (see FIG. 2C, 206).
FIG. 2C is a drawing of a blood transfer part comprising a blood sample capillary tube and compressible chamber in side view (top) and cross section view (bottom). 203: blood sample capillary tube small end; 204: blood sample capillary tube center portion; 205: blood sample capillary tube large end; 206: compressible chamber; 207: inner diameter of blood sample capillary tube small end; 208: inner diameter of blood sample capillary tube large end.

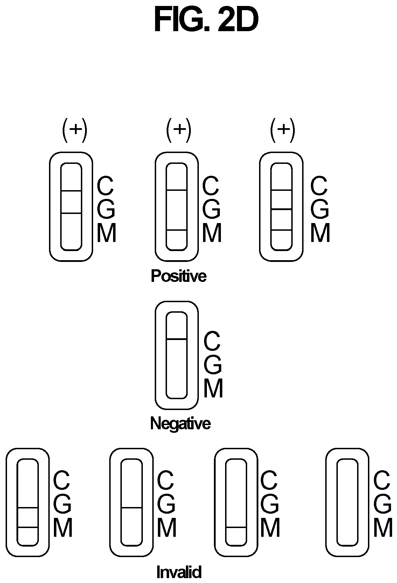

FIG. 2D is an enlarged view of the control (C), IgG (G) test line, and IgM (M) line of the test results viewing window 211 showing the combinations of visible lines that indicate a positive (top three diagrams), a negative (middle diagram), or an invalid (bottom four diagrams) test result.

It is to be understood that the figures are not necessarily drawn to scale, nor are the objects in the figures necessarily drawn to scale in relationship to one another. The figures are depictions that are intended to bring clarity and understanding to various embodiments of apparatuses, systems, and methods disclosed herein. Wherever possible, the same reference numbers will be used throughout the drawings to refer to the same or like parts. Moreover, it should be appreciated that the drawings are not intended to limit the scope of the present teachings in any way.

### DETAILED DESCRIPTION

Provided herein is technology relating to point of care assays and particularly, but not exclusively, to devices, methods, and systems for detecting pathogen antigens and/or antibodies specific for pathogen antigens in patient samples.

In this detailed description of the various embodiments, for purposes of explanation, numerous specific details are set forth to provide a thorough understanding of the embodiments disclosed. One skilled in the art will appreciate, however, that these various embodiments may be practiced with or without these specific details. In other instances, structures and devices are shown in block diagram form. Furthermore, one skilled in the art can readily appreciate that the specific sequences in which methods are presented and performed are illustrative and it is contemplated that the sequences can be varied.

Unless defined otherwise, all technical and scientific terms used herein have the same meaning as is commonly understood by one of ordinary skill in the art to which the various embodiments described herein belongs. The section headings used herein are for organizational purposes only and are not to be construed as limiting the described subject matter in any way.

### Definitions

To facilitate an understanding of the present technology, a number of terms and phrases are defined below. Additional definitions are set forth throughout the detailed description.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined.

In addition, as used herein, the term "or" is an inclusive "or" operator and is equivalent to the term "and/or" unless the context clearly dictates otherwise. The term "based on" is not exclusive and allows for being based on additional factors not described, unless the context clearly dictates otherwise. In addition, throughout the specification, the meaning of "a", "an", and "the" include plural references. The meaning of "in" includes "in" and "on."

As used herein, the terms "about", "approximately", "substantially", and "significantly" are understood by persons of ordinary skill in the art and will vary to some extent on the context in which they are used. If there are uses of these terms that are not clear to persons of ordinary skill in the art given the context in which they are used, "about" and "approximately" mean plus or minus less than or equal to 10% of the particular term and "substantially" and "significantly" mean plus or minus greater than 10% of the particular term.

As used herein, disclosure of ranges includes disclosure of all values and further divided ranges within the entire range, including endpoints and sub-ranges given for the ranges.

As used herein, the suffix "-free" refers to an embodiment of the technology that omits the feature of the base root of the word to which "-free" is appended. That is, the term "X-free" as used herein means "without X", where X is a feature of the technology omitted in the "X-free" technology. For example, a "calcium-free" composition does not comprise calcium, a "mixing-free" method does not comprise a mixing step, etc.

Although the terms "first", "second", "third", etc. may be used herein to describe various steps, elements, compositions, components, regions, layers, and/or sections, these steps, elements, compositions, components, regions, layers, and/or sections should not be limited by these terms, unless otherwise indicated. These terms are used to distinguish one step, element, composition, component, region, layer, and/or section from another step, element, composition, component, region, layer, and/or section. Terms such as "first", "second", and other numerical terms when used herein do not imply a sequence or order unless clearly indicated by the context. Thus, a first step, element, composition, component, region, layer, or section discussed herein could be termed a second step, element, composition, component, region, layer, or section without departing from technology.

As used herein, the word "presence" or "absence" (or, alternatively, "present or "absent") is used in a relative sense to describe the amount or level of a particular entity (e.g., an analyte). For example, when an analyte is said to be "present" in a test sample, it means the level or amount of this analyte is above a pre-determined threshold; conversely, when an analyte is said to be "absent" in a test sample, it means the level or amount of this analyte is below a pre-determined threshold. The pre-determined threshold may be the threshold for detectability associated with the particular test used to detect the analyte or any other threshold. When an analyte is "detected" in a sample it is "present" in the sample; when an analyte is "not detected" it is "absent" from the sample. Further, a sample in which an analyte is "detected" or in which the analyte is "present" is a sample that is "positive" for the analyte. A sample in which an analyte is "not detected" or in which the analyte is "absent" is a sample that is "negative" for the analyte.

As used herein, an "increase" or a "decrease" refers to a detectable (e.g., measured) positive or negative change, respectively, in the value of a variable relative to a previously measured value of the variable, relative to a pre-established value, and/or relative to a value of a standard control. An increase is a positive change preferably at least 10%, more preferably 50%, still more preferably 2-fold, even more preferably at least 5-fold, and most preferably at least 10-fold relative to the previously measured value of the variable, the pre-established value, and/or the value of a standard control. Similarly, a decrease is a negative change preferably at least 10%, more preferably 50%, still more preferably at least 80%, and most preferably at least 90% of the previously measured value of the variable, the pre-established value, and/or the value of a standard control. Other terms indicating quantitative changes or differences, such as "more" or "less," are used herein in the same fashion as described above.

As used herein, a "system" refers to a plurality of real and/or abstract components operating together for a common purpose. In some embodiments, a "system" is an integrated assemblage of hardware and/or software components. In some embodiments, each component of the system interacts with one or more other components and/or is related to one or more other components. In some embodiments, a system refers to a combination of components and software for controlling and directing methods.

As used herein, the term "analyte" refers to a compound or composition to be detected and/or measured by specific binding to a ligand, receptor, or enzyme (e.g., an antibody or antigen). In some embodiments, the analyte is a protein or a nucleic acid. In some embodiments, the analyte is an antigen, an antibody, and/or a receptor. In some embodiments, the analyte is a fragment of an antigen, an antibody, and/or a receptor. In some embodiments, the analyte is an analyte analogue or an analyte derivative (e.g., an analyte altered by chemical or biological methods). In some embodiments, an analyte is an epitope. As described herein, in some embodiments, the analyte is from a pathogen.

As used herein the term "pathogen" refers to an organism, including a microorganism, which causes disease in another organism (e.g., animals (e.g., humans) and plants) by directly infecting the other organism, or by producing agents that causes disease in another organism (e.g., bacteria that produce pathogenic toxins and the like). As used herein, pathogens include, but are not limited to prokaryotes and eukaryotes (e.g., any member of the Bacteria, Archaea, and/or Eukaryota) and thus the term includes pathogenic organisms described as bacteria, eukaryotes, archaeabacteria, protozoa, fungi, nematodes, viroids and viruses, or any combination thereof, wherein a pathogen is capable, either by itself or in concert with another pathogen, of eliciting disease in vertebrates including but not limited to mammals, and including but not limited to humans. As used herein, the term "pathogen" also encompasses microorganisms which may not ordinarily be pathogenic in a non-immunocompromised host. Specific nonlimiting examples of viral pathogens include Herpes simplex virus (HSV)1, HSV2, Epstein Barr virus (EBV), cytomegalovirus (CMV), human Herpes virus (HHV) 6, HHV7, HHV8, Varicella zoster virus (VZV), hepatitis C, hepatitis B, adenovirus, Eastern Equine Encephalitis Virus (EEEV), West Nile virus (WNE), JC virus (JCV), BK virus (BKV), MERS, SARS, SARS-CoV-2, influenza virus, Zika virus, Chikungunya virus, Aura virus, Bebaru virus, Cabassou virus, Dengue virus, Fort morgan virus, Getah virus, Kyzylagach virus, Mayoaro virus, Middleburg virus, Mucambo virus, Ndumu virus, Pixuna virus, Tonate virus, Triniti virus, Una virus, Western equine encephalomyelitis virus, Whataroa virus, Sindbis virus (SIN), Semliki forest virus (SFV), Venezuelan equine encephalomyelitis virus (VEE), Ross River virus, human immunodeficiency virus (HIV-1, HIV-2), and HTLV (HTLV-1, HTLV-2, HTLV-3, and HTLV-4). See, e.g., Strauss and Strauss, Microbiol. Rev., 58:491-562 (1994), incorporated herein by reference.

As used herein, the term "microorganism" includes prokaryotic and eukaryotic microbial species from the Domains of Archaea, Bacteria, and Eucarya, the latter including yeast and filamentous fungi, protozoa, algae, or higher Protista. The terms "microbial cells" and "microbes" are used interchangeably with the term microorganism.

The terms "bacteria" and "bacterium" refer to prokaryotic organisms of the domain Bacteria in the three-domain system (see, e.g., Woese CR, et al., Proc Natl Acad Sci USA 1990, 87: 4576 - 79). It is intended that the terms encompass all microorganisms considered to be bacteria including Mycobacterium, Mycoplasma, Chlamydia, Actinomyces, Streptomyces, and Rickettsia. All forms of bacteria are included within this definition including cocci, bacilli, spirochetes, spheroplasts, protoplasts, etc. In some embodiments, bacteria are capable of causing disease and product degradation or spoilage. Accordingly, "Bacteria", or "Eubacteria", refers to a domain of prokaryotic organisms. Bacteria include at least 11 distinct groups as follows: (1) Gram-positive (gram+) bacteria, of which there are two major subdivisions: (i) high G+C group (Actinomycetes, Mycobacteria, Micrococcus, others) (ii) low G+C group (Bacillus, Clostridia, Lactobacillus, Staphylococci, Streptococci, Mycoplasmas); (2) Proteobacteria, e.g., Purple photosynthetic+non-photosynthetic Gram-negative bacteria (includes most "common" Gram-negative bacteria); (3) Cyanobacteria, e.g., oxygenic phototrophs; (4) Spirochetes and related species; (5) Planctomyces; (6) Bacteroides, Flavobacteria; (7) Chlamydia; (8) Green sulfur bacteria; (9) Green non-sulfur bacteria (also anaerobic phototrophs); (10) Radioresistant micrococci and relatives; (11) Thermotoga and Thermosipho thermophiles.

"Gram-negative bacteria" include cocci, nonenteric rods, and enteric rods. The genera of Gram-negative bacteria include, for example, Neisseria, Spirillum, Pasteurella, Brucella, Yersinia, Francisella, Haemophilus, Bordetella, Escherichia, Salmonella, Shigella, Klebsiella, Proteus, Vibrio, Pseudomonas, Bacteroides, Acetobacter, Aerobacter, Agrobacterium, Azotobacter, Spirilla, Serratia, Vibrio, Rhizobium, Chlamydia, Rickettsia, Treponema, and Fusobacterium.

"Gram-positive bacteria" include cocci, nonsporulating rods, and sporulating rods. The genera of Gram-positive bacteria include, for example, Actinomyces, Bacillus, Clostridium, Corynebacterium, Erysipelothrix, Lactobacillus, Listeria, Mycobacterium, Myxococcus, Nocardia, Staphylococcus, Streptococcus, and Streptomyces.

As used herein, the term "antibody" refers to an immunoglobulin, an immunoglobulin derivative, and/or an immunoglobulin fragment. An antibody comprises an area on its surface or in a cavity that specifically binds to a particular spatial and/or polar organization of another molecule. The antibody can be monoclonal or polyclonal and can be prepared by techniques that are well known in the art such as, for example, immunization of a host and collection of sera or hybrid cell line technology. Accordingly, the term "antibody" refers to an immunoglobulin, derivatives thereof that maintain specific binding ability, and proteins having a binding domain that is homologous or substantially and/or effectively homologous to an immunoglobulin binding domain. These proteins may be derived from natural sources or partly or wholly synthetically produced. The antibody may be a member of any immunoglobulin class, including any of the human classes: IgG, IgM, IgA, IgD, and IgE. The basic antibody structural unit is known to comprise a tetramer. Each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Light chains are classified as either kappa or lambda. Heavy chains are classified as gamma, mu, alpha, delta, or epsilon, and define the antibody isotype as IgG, IgM, IgA, IgD, or IgE, respectively. Within light and heavy chains, the variable and constant regions are joined by a "J" region of about 12 or more amino acids, with the heavy chain also including a "D" region of about 10 more amino acids. (See generally, Fundamental Immunology (See, e.g., Paul, Fundamental Immunology, 3rd Ed., 1993, Raven Press, New York). The variable regions of each light/heavy chain pair form the antibody binding site. The chains all exhibit the same general structure of relatively conserved framework regions (FR) joined by three hypervariable regions, also called complementarily determining regions or CDRs. The CDRs from the two chains of each pair are aligned by the framework regions, enabling binding to a specific epitope. CDR and FR residues are delineated according to the standard sequence definition of Kabat et al. (5th ed., 1991) Sequences of Proteins of Immunological Interest (National Institutes of Health publication 91-3242). An alternative structural definition has been proposed by Chothia et al. (1987) J. Mol. Biol. 196: 901-917; (1989) Nature 342: 878-883; and (1989) J. Mol. Biol. 186: 651-663.

As used herein, the term "antibody fragment" refers to any derivative of an antibody that comprises an amino acid sequence that is less than a full-length antibody amino acid sequence. In exemplary embodiments, the antibody fragment retains at least a significant portion of the specific binding ability of the full-length antibody. Examples of antibody fragments include, but are not limited to, Fab, Fab', F(ab')₂, scFv, Fv, dsFv diabody, and Fd fragments. The antibody fragment may be produced by any means. For instance, the antibody fragment may be enzymatically or chemically produced by fragmentation of an intact antibody, it may be recombinantly produced from a gene encoding the partial antibody sequence, or it may be wholly or partially synthetically produced. For example, in some embodiments, the term Fab fragment may refer to a binding fragment resulting from papain cleavage of an intact antibody and the terms Fab' and F(ab')₂ may refer to binding fragments of intact antibodies generated by pepsin cleavage. As used herein, the term "Fab" is used to refer generically to double chain binding fragments of intact antibodies having at least substantially complete light and heavy chain variable domains sufficient for antigen-specific bindings and parts of the light and heavy chain constant regions sufficient to maintain association of the light and heavy chains. Usually, Fab fragments are formed by complexing a full-length or substantially full-length light chain with a heavy chain comprising the variable domain and at least the CH1 domain of the constant region The antibody fragment may optionally be a single chain antibody fragment. Alternatively, the fragment may comprise multiple chains that are linked together, for instance, by disulfide linkages. The fragment may also optionally be a multimolecular complex. A functional antibody fragment will typically comprise at least about 50 amino acids and more typically will comprise at least about 200 amino acids.

As used herein, the terms "specifically binds to" or "specifically immunoreactive with", e.g., when referring to an antibody, antibody fragment, antigen, or other binding moiety, refers to a binding reaction that is determinative of the presence of a target analyte in the presence of a heterogeneous population of proteins and/or other biologics. Thus, under designated assay conditions, the specified binding moieties bind preferentially to a particular target analyte and do not bind in a significant amount to other components present in a test sample. Specific binding to a target antigen under such conditions may require a binding moiety that is selected for its specificity for a particular target analyte. A variety of immunoassay formats may be used to select antibodies that are specifically immunoreactive with a particular protein. For example, solid-phase ELISA immunoassays are routinely used to select monoclonal antibodies specifically immunoreactive with an antigen. See Harlow and Lane (1988) Antibodies, A Laboratory Manual, Cold Spring Harbor Publications, New York, for a description of immunoassay formats and conditions that can be used to determine specific immunoreactivity. Typically, a specific or selective reaction is at least twice background signal or noise and more typically more than 10 to 100 times background. Specific binding between an antibody or other binding agent and an antigen generally means a binding affinity of at least 10⁶ M⁻¹. Preferred binding agents bind with affinities of at least about 10⁷ M⁻¹, and preferably 10⁸ M⁻¹ to 10⁹ M⁻¹ or 10¹⁰ M⁻¹.

As used herein, the term "epitope" refers to an antigenic determinant that is capable of specific binding to an antibody. Epitopes usually comprise chemically active surface groupings of molecular moieties, e.g., as amino acids or sugar side chains, and usually have specific three-dimensional structural characteristics and/or specific charge characteristics. Conformational and nonconformational epitopes are distinguished in that the binding to the former but not the latter is lost in the presence of denaturing solvents. Epitopes can include non-contiguous amino acids, as well as contiguous amino acids.

As used herein, the term "sample" refers to any sample comprising a pathogen or a part or component thereof or that potentially comprises a pathogen or a part or component thereof. Accordingly, the term "sample" refers to a material to be tested for the presence or amount of an analyte, e.g., a pathogen or a part or component thereof. Preferably, a sample is a fluid sample, preferably a liquid sample. For example, a sample may be a bodily fluid such as blood, serum, plasma, ocular fluid, urine, mucus, semen, nasopharyngeal swab fluid, throat swab, tears, sweat, or saliva. Viscous liquid, semi-solid, or solid specimens may be used to create liquid solutions, eluates, suspensions, or extracts that can be samples. For example, throat or genital swabs may be suspended in a liquid solution to make a sample.

As used herein, the term "test strip" or, equivalently, "lateral flow assay test strip" can include one or more bibulous or non-bibulous materials. If a test strip comprises more than one material, the one or more materials are preferably in fluid communication. One material of a test strip may be overlaid on another material of the test strip, such as for example, filter paper overlaid on nitrocellulose. Alternatively or in addition, a test strip may include a region comprising one or more materials followed by a region comprising one or more different materials. In this case, the regions are in fluid communication and may or may not partially overlap one another. Suitable materials for test strips include, but are not limited to, materials derived from cellulose, such as filter paper, chromatographic paper, nitrocellulose, and cellulose acetate, as well as materials made of glass fibers, nylon, dacron, PVC, polyacrylamide, cross-linked dextran, agarose, polyacrylate, ceramic materials, and the like. The material or materials of the test strip may optionally be treated to modify their capillary flow characteristics or the characteristics of the applied sample. For example, the sample application region of the test strip may be treated with buffers to correct the pH, salt concentration, or specific gravity of an applied sample to optimize test conditions.

The material or materials can be a single structure such as a sheet cut into strips or it can be several strips or particulate material bound to a support or solid surface such as found, for example, in thin-layer chromatography and may have an absorbent pad either as an integral part or in liquid contact. The material can also be a sheet having lanes thereon, capable of spotting to induce lane formation, wherein a separate assay can be conducted in each lane. The material can have a rectangular, circular, oval, triangular, or other shape provided that there is at least one direction of traversal of a test solution by capillary migration. Other directions of traversal may occur such as in an oval or circular piece contacted in the center with the test solution. However, the main consideration is that there be at least one direction of flow to a predetermined site.

The support for the test strip, where a support is desired or necessary, will normally be water insoluble, frequently non-porous and rigid but may be elastic, usually hydrophobic, and porous and usually will be of the same length and width as the strip but may be larger or smaller. The support material can be transparent, and, when a test device of the present technology is assembled, a transparent support material can be on the side of the test strip that can be viewed by the user, such that the transparent support material forms a protective layer over the test strip where it may be exposed to the external environment, such as by an aperture in the front of a test device. A wide variety of non-mobilizable and non-mobilizable materials, both natural and synthetic, and combinations thereof, may be employed provided only that the support does not interfere with the capillary action of the material or materials, or non-specifically bind assay components, or interfere with the signal producing system. Illustrative polymers include polyethylene, polypropylene, poly(4-methylbutene), polystyrene, polymethacrylate, polyethylene terephthalate), nylon, poly(vinyl butyrate), glass, ceramics, metals, and the like. Elastic supports may be made of polyurethane, neoprene, latex, silicone rubber and the like.

As used herein, the term "control zone" or "control line" is a region of a test strip in which a label can be observed to shift location, appear, change color, or disappear to indicate that an assay performed correctly. Detection or observation of the control zone (e.g., of a control line) may be done by any convenient means, depending upon the particular choice of label, especially, for example but not limited to, visually, fluorescently, by reflectance, radiographically, and the like. As will be described, the label may or may not be applied directly to the control zone, depending upon the design of the control being used.

As used herein, the term "label" refers to any molecule bound to a specific binding member that can produce a detectable signal. In the present invention, the label may be inert and provide a signal by concentrating in the detection zone, it may serve solely as a binding site for a member of the signal producing system, or it may spontaneously produce a detectable signal or may produce a detectable signal in conjunction with a signal producing system. The label may be isotopic or nonisotopic. In some embodiments, the label comprises a gold colloid, latex beads, a dye, a fluorescent moiety, or other detectable entity.

As used herein, the term "proximal end" refers to the end of a test device or test strip that includes the sample application aperture of the test device and/or the sample application zone of the test strip.

As used herein, the term "reagent zone" refers to a region of a test strip where reagent is provided. The reagent zone can be on a reagent pad, a separate segment of bibulous or non-bibulous material included on the test strip, or it can be a region of a bibulous or non-bibulous material of a test strip that also includes other zones, such as an analyte detection zone. The reagent zone can carry a detectable label, which may be a direct or indirect label. Preferably the reagent is provided in a form that is immobile in the dry state and mobile in the moist state. A reagent can be a specific binding member, an analyte or analyte analog, an enzyme, a substrate, indicators, components of a signal producing system, chemicals or compounds such as buffering agents, reducing agents, chelators, surfactants, etc., that contribute to the function of the test strip assay.

As used herein, the term "sample application aperture" refers to the portion of a test device where an opening in the test device provides access to the sample application zone of the test strip.

As used herein, the term "sample application zone" is the portion of a test strip where sample is applied. In some embodiments, a "sample pad" comprises a sample application zone.

As used herein, the term "specific binding member" refers to one of two different molecules having an area on the surface or in a cavity that specifically binds to and is thereby defined as complementary with a particular spatial and polar organization of the other, second molecule. The members of the specific binding pair are referred to as ligand and receptor (antiligand). These will usually be members of an immunological pair such as antigen-antibody, although other specific binding pairs, e.g., biotin-avidin, hormone-hormone receptor, nucleic acid duplexes, IgG-protein A, DNA-DNA, DNA-RNA, and the like, are not immunological pairs but are included in the definition. In the case of binding pairs such as avidin-biotin, reagent can be labeled with one member of this pair and a detection zone can include the other member of this pair in a capture type assay. Other general types of assays using avidin-biotin pairs or binding pairs of this type are known in the art. An antibody (e.g., a labeled antibody) can be used as a reagent for the detection of an antigen that binds with or specifically binds with such an antibody. An antigen or epitope (e.g., a labeled antigen) can be used as a reagent for the detection of antibodies that bind with or specifically bind with such an antigen or epitope.

As used herein, the term "test results zone" is a region of a test strip that provides a detectable signal indicating the presence of the analyte. The test results zone can include an immobilized binding reagent specific for an analyte ("specific binding member") and/or an enzyme that reacts with the analyte. A test results zone can include one or more analyte detection zones, e.g., a "test line". Other substances that may allow or enhance detection of the analyte, such as substrates, buffers, salts, may also be provided in the test results zone. One or more members of a signal producing system may be bound directly or indirectly to the detection zone. A test results zone can optionally include one or more control zones (e.g., a "control line") that provide indication that the test has been performed properly.

The terms "identical" or percent "identity," in the context of two or more nucleic acids or polypeptide sequences, refer to two or more sequences or subsequences that are the same or have a specified percentage of nucleotides or amino acid residues that are the same, when compared and aligned for maximum correspondence, as measured using one of the following sequence comparison algorithms or by visual inspection. The phrase "substantially identical," in the context of two nucleic acids, refers to two or more sequences or subsequences that have at least 80%, preferably 85%, most preferably 90-95% nucleotide identity, when compared and aligned for maximum correspondence, as measured using one of the following sequence comparison algorithms or by visual inspection. For amino acid sequences, "substantially identical" refers to two or more sequences or subsequences that have at least 60% identity, preferably 75% identity, and more preferably 90-95% identify, when compared and aligned for maximum correspondence, as measured using one of the following sequence comparison algorithms or by visual inspection. Preferably, the substantial identity exists over a region of the nucleic acid or amino acid sequences that is at least about 10 residues in length, more preferably over a region of at least about 20 residues, and most preferably the sequences are substantially identical over at least about 100 residues. In a most preferred embodiment, the sequences are substantially identical over the entire length of the specified regions (e.g., coding regions).

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters. Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally, Current Protocols in Molecular Biology, F. M. Ausubel et al., eds., Current Protocols, a joint venture between Greene Publishing Associates, Inc. and John Wiley & Sons, Inc., (1995 Supplement) (Ausubel)).

Examples of algorithms that are suitable for determining percent sequence identity and sequence similarity are the BLAST and BLAST 2.0 algorithms, which are described in Altschul et al. (1990) J. Mol. Biol. 215: 403-410 and Altschuel et al. (1977) Nucleic Acids Res. 25: 3389-3402, respectively. Software for performing BLAST analyses is publicly available through the National Center for Biotechnology Information (ncbi.nlm.nih.gov). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul et al, supra). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix (see Henikoff & Henikoff, Proc. Natl. Acad. Sci. USA 89:10915 (1989)).

A further indication that two nucleic acids or polypeptides are substantially identical is that the polypeptide encoded by the first nucleic acid is immunologically cross-reactive with the polypeptide encoded by the second nucleic acid, as described below. Thus, a polypeptide is typically substantially identical to a second polypeptide, for example, where the two peptides differ only by conservative substitutions. Another indication that two nucleic acid sequences are substantially identical is that the two molecules hybridize to each other under stringent conditions, as described below.

Thus, the terms "variant" and "mutant" when used in reference to a nucleotide sequence refer to a nucleic acid sequence that differs by one or more nucleotides from another, usually related nucleotide acid sequence. A "variation" is a difference between two different nucleotide sequences; typically, one sequence is a reference sequence.

The terms "variant" and "mutant" when used in reference to a polypeptide refer to an amino acid sequence that differs by one or more amino acids from another, usually related polypeptide. The variant may have "conservative" changes, wherein a substituted amino acid has similar structural or chemical properties. One type of conservative amino acid substitution refers to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine. More rarely, a variant may have "non-conservative" changes (e.g., replacement of a glycine with a tryptophan). Similar minor variations may also include amino acid deletions or insertions (i.e., additions), or both. Guidance in determining which and how many amino acid residues may be substituted, inserted or deleted without abolishing biological activity may be found using computer programs well known in the art, for example, DNAStar software. Variants can be tested in functional assays. Preferred variants have less than 10%, and preferably less than 5%, and still more preferably less than 2% changes (whether substitutions, deletions, and so on).

Accordingly, as used herein, the term "conservatively modified variations" of a particular polynucleotide sequence refers to those polynucleotides that encode identical or essentially identical amino acid sequences, or where the polynucleotide does not encode an amino acid sequence, to essentially identical sequences. Because of the degeneracy of the genetic code, a large number of functionally identical nucleic acids encode any given polypeptide. For instance, the codons CGU, CGC, CGA, CGG, AGA, and AGG all encode the amino acid arginine. Thus, at every position where an arginine is specified by a codon, the codon can be altered to any of the corresponding codons described without altering the encoded polypeptide. Such nucleic acid variations are "silent substitutions" or "silent variations," which are one species of "conservatively modified variations." Every polynucleotide sequence described herein which encodes a polypeptide also describes every possible silent variation, except where otherwise noted. Thus, silent substitutions are an implied feature of every nucleic acid sequence which encodes an amino acid. One of skill will recognize that each codon in a nucleic acid (except AUG, which is ordinarily the only codon for methionine and UGG, the only codon for tryptophan) can be modified to yield a functionally identical molecule by standard techniques. In some embodiments, the nucleotide sequences that encode the enzymes are preferably optimized for expression in a particular host cell (e.g., yeast, mammalian, plant, fungal, and the like) used to produce the enzymes.

Similarly, "conservative amino acid substitutions," in one or a few amino acids in an amino acid sequence are substituted with different amino acids with highly similar properties are also readily identified as being highly similar to a particular amino acid sequence, or to a particular nucleic acid sequence which encodes an amino acid.

Individual substitutions, deletions or additions which alter, add or delete a single amino acid or a small percentage of amino acids (typically less than 5%, more typically less than 1%) in an encoded sequence are "conservatively modified variations" where the alterations result in the substitution of an amino acid with a chemically similar amino acid. Conservative substitution tables providing functionally similar amino acids are well known in the art. See, e.g., Creighton (1984) Proteins, W.H. Freeman and Company.

The terms "protein" and "polypeptide" refer to compounds comprising amino acids joined via peptide bonds and are used interchangeably. Conventional one and three-letter amino acid codes are used herein as follows - Alanine: Ala, A; Arginine: Arg, R; Asparagine: Asn, N; Aspartate: Asp, D; Cysteine: Cys, C; Glutamate: Glu, E; Glutamine: Gln, Q; Glycine: Gly, G; Histidine: His, H; Isoleucine: Ile, I; Leucine: Leu, L; Lysine: Lys, K; Methionine: Met, M; Phenylalanine: Phe, F; Proline: Pro, P; Serine: Ser, S; Threonine: Thr, T; Tryptophan: Trp, W; Tyrosine: Tyr, Y; Valine: Val, V. As used herein, the codes Xaa and X refer to any amino acid.

It is well known that DNA (deoxyribonucleic acid) is a chain of nucleotides consisting of 4 types of nucleotides; A (adenine), T (thymine), C (cytosine), and G (guanine), and that RNA (ribonucleic acid) is comprised of 4 types of nucleotides; A, U (uracil), G, and C. It is also known that all of these 5 types of nucleotides specifically bind to one another in combinations called complementary base pairing. That is, adenine (A) pairs with thymine (T) (in the case of RNA, however, adenine (A) pairs with uracil (U)), and cytosine (C) pairs with guanine (G), so that each of these base pairs forms a double strand.

The nomenclature used to describe variants of nucleic acids or proteins specifies the type of mutation and base or amino acid changes. For a nucleotide substitution (e.g., 76A>T), the number is the position of the nucleotide from the 5' end, the first letter represents the wild type nucleotide, and the second letter represents the nucleotide which replaced the wild type. In the given example, the adenine at the 76th position was replaced by a thymine. If it becomes necessary to differentiate between mutations in genomic DNA, mitochondrial DNA, complementary DNA (cDNA), and RNA, a simple convention is used. For example, if the 100th base of a nucleotide sequence is mutated from G to C, then it would be written as g.100G>C if the mutation occurred in genomic DNA, m.100G>C if the mutation occurred in mitochondrial DNA, c.100G>C if the mutation occurred in cDNA, or r.100g>c if the mutation occurred in RNA.

For amino acid substitution (e.g., D111E), the first letter is the one letter code of the wild type amino acid, the number is the position of the amino acid from the N-terminus, and the second letter is the one letter code of the amino acid present in the mutation. Nonsense mutations are represented with an X for the second amino acid (e.g. D111X). For amino acid deletions (e.g. ΔF508, F508del), the Greek letter Δ (delta) or the letters "del" indicate a deletion. The letter refers to the amino acid present in the wild type and the number is the position from the N terminus of the amino acid where it is present in the wild type. Intronic mutations are designated by the intron number or cDNA position and provide either a positive number starting from the G of the GT splice donor site or a negative number starting from the G of the AG splice acceptor site. g.3' +7G>C denotes the G to C substitution at nt +7 at the genomic DNA level. When the full-length genomic sequence is known, the mutation is best designated by the nucleotide number of the genomic reference sequence. See den Dunnen & Antonarakis, "Mutation nomenclature extensions and suggestions to describe complex mutations: a discussion". Human Mutation 15: 7-12 (2000); Ogino S, et al., "Standard Mutation Nomenclature in Molecular Diagnostics: Practical and Educational Challenges", J. Mol. Diagn. 9(1): 1-6 (February 2007).

As used herein, the one-letter codes for amino acids refer to standard IUB nomenclature as described in "IUPAC-IUB Nomenclature of Amino Acids and Peptides" published in Biochem. J., 1984, 219, 345-373; Eur. J. Biochem., 1984, 138, 9-37; 1985, 152, 1; Internat. J. Pept. Prot. Res., 1984, 24, following p 84; J. Biol. Chem., 1985, 260, 14-42; Pure Appl. Chem., 1984, 56, 595-624; Amino Acids and Peptides, 1985, 16, 387-410; and in Biochemical Nomenclature and Related Documents, 2nd edition, Portland Press, 1992, pp 39-67, each of which is incorporated herein by reference. The following degenerate codes may be used for nucleotide bases: R (G or A), Y (T/U or C), M (A or C), K (G or T/U), S (G or C), W (A or T/U), B (G or C or T/U), D (A or G or T/U), H (A or C or T/U), V (A or G or C), or N (A or G or C or T/U), gap (-).

As used herein, the term "coupled" refers to two or more components that are secured, by any suitable means, together. Accordingly, in some embodiments, the statement that two or more parts or components are "coupled" shall mean that the parts are joined or operate together either directly or indirectly, e.g., through one or more intermediate parts or components. As used herein, "directly coupled" means that two elements are directly in contact with each other. As used herein, "fixedly coupled" or "fixed" means that two components are coupled so as to move as one while maintaining a constant orientation relative to each other. Accordingly, when two elements are coupled, all portions of those elements are coupled. A description, however, of a specific portion of a first element being coupled to a second element, e.g., an axle first end being coupled to a first wheel, means that the specific portion of the first element is disposed closer to the second element than the other portions thereof. Further, an object resting on another object held in place only by gravity is not "coupled" to the lower object unless the upper object is otherwise maintained substantially in place. That is, for example, a book on a table is not coupled thereto, but a book glued to a table is coupled thereto.

As used herein, the term "fluid communication" refers to connected fluid elements comprising a fluid interface among and between the elements so that fluid can transfer from one element to the other. Accordingly, the term "fluid communication" as used herein refers to two components, chambers, or regions containing a fluid, where the components, chambers, or regions are connected together (e.g., by a line, a pipe, or tubing) so that a fluid can flow between the two chambers, components, or regions. Therefore, two components that are in "fluid communication" can, for example, be connected together by a line between the two chambers, such that a fluid can flow freely between the two chambers.

As used herein, the term "metered" refers to a reproducibly (e.g., within errors associated with a measurement) measured and quantified amount (e.g., volume) of a substance (e.g., a sample) that is provided from a larger amount (e.g., volume) of the substance (e.g., sample). Accordingly, a "metered sample" is a known and measured amount (e.g., volume) of a sample that is reproducible and thus a "metered sample" of a larger amount of the sample is predicted to be the same (e.g., substantially and/or essentially the same) amount of the sample each time the metered sample is produced. Thus the amount (e.g., volume) of a substance in a future metered sample is expected to have an amount (e.g., volume) that is the same (e.g., substantially and/or essentially the same) as is provided in a present metered sample.

As used herein, the term "configured" refers to a component, module, system, subsystem, etc. that is constructed to carry out the indicated function.

### Lateral Flow Assays

Lateral flow assays are used in hospital, clinical, and home settings (e.g., in a self-test device, e.g., for performing a method comprising self-administering a test to detect a pathogen by contacting a sample to the self-test device). These devices are used to test for a variety of analytes, such as drugs of abuse, hormones, proteins, pathogens (e.g., and antigens thereof), plasma components, antibodies, etc. Lateral flow assays are generally provided in a device (e.g., an assay device) comprising a lateral flow assay test strip (e.g., nitrocellulose or filter paper), a sample application area (e.g., sample pad), a test results area (e.g., a test line), an optional control results area (e.g., a control line), and an analyte-specific binding reagent that is bound to a detectable label (e.g., a colored particle or an enzyme detection system). See, e.g., U.S. Pat. Nos. 6,485,982; 6,187,598; 5,622,871; 6,565,808; and 6,809,687; and U.S. Pat. App. Ser. No. 10/717,082. See, e.g., FIG. 1.

In particular, embodiments provide assays for detecting a pathogen in a sample. In some embodiments, embodiments relate to detecting antibodies (e.g., IgG and/or IgM) against a pathogen a sample. In some embodiments, the technology relates to assay devices that are suitable for use in the home, clinic, or hospital, and that are intended to give an analytical result that is rapid with a minimum degree of skill and involvement from the user. In some embodiments, use of the devices described herein involves methods in which a user performs a sequence of operations to provide an observable test result.

In some embodiments, the technology relates to an assay device comprising a lateral flow assay test strip (e.g., a reagent-impregnated lateral flow assay test strip) to provide a specific binding assay, e.g., an immunoassay. See, e.g., FIG. 1. In some embodiments, a sample is applied to one portion of the lateral flow assay test strip and is allowed to permeate through the lateral flow assay test strip material, usually with the aid of an eluting solvent such as water and/or a suitable buffer (e.g., optionally comprising a detergent). In so doing, the sample progresses into or through a detection zone in the lateral flow assay test strip wherein a specific binding reagent (e.g., an antibody) for an analyte (e.g., a pathogen or a portion or component thereof, an anti-pathogen antibody (e.g., an IgG and/or an IgM specific for the pathogen) suspected of being in the sample is immobilized. Analyte present in the sample can therefore become bound within the detection zone. The extent to which the analyte becomes bound in that zone can be determined with the aid of labelled reagents that can also be incorporated in the lateral flow assay test or applied thereto subsequently.

In some embodiments, the assay device comprises a hollow casing constructed of moisture-impervious solid material containing a dry porous carrier that communicates directly or indirectly with the exterior of the casing such that a liquid test sample can be applied to the porous carrier. In some embodiments, the assay device also comprises a labelled specific binding reagent for an analyte and the labelled specific binding reagent is freely mobile within the porous carrier when in the moist state. In some embodiments, the assay device comprises unlabeled specific binding reagent for the same analyte and the unlabeled reagent is permanently immobilized in a detection zone on the carrier material and is therefore not mobile in the moist state. The relative positioning of the labelled reagent and detection zone being such that liquid sample applied to the assay device can pick up labelled reagent and thereafter permeate into the detection zone and the assay device provides the extent (if any) to which the labelled reagent becomes in the detection zone to be observed.

Another embodiment of the technology relates to an assay device that comprises a porous solid phase material carrying in a first zone a labelled reagent that is retained in the first zone while the porous material is in the dry state but is free to migrate through the porous material when the porous material is moistened, for example, by the application of an aqueous liquid sample suspected of containing the analyte. In some embodiments, the porous material comprises in a second zone, which is spatially distinct from the first zone, an unlabeled specific binding reagent having specificity for the analyte and which is capable of participating with the labelled reagent in either a "sandwich" or a "competition" reaction. The unlabeled specific binding reagent is firmly immobilized on the porous material such that it is not free to migrate when the porous material is in the moist state.

In some embodiments, the technology also provides an analytical method in which an assay device as described herein is contacted with an aqueous liquid sample suspected of containing the analyte, such that the sample permeates by capillary action through the porous solid phase material via the first zone into the second zone and the labelled reagent migrates therewith from the first zone to the second zone, the presence of analyte in the sample being determined by observing the extent (if any) to which the labelled reagent becomes bound in the second zone.

In some embodiments, the labelled reagent is a specific binding partner for the analyte. The labelled reagent, the analyte (if present), and the immobilized unlabeled specific binding reagent cooperate together in a "sandwich" reaction. This results in the labelled reagent being bound in the second zone if analyte is present in the sample. In a sandwich format, the two binding reagents have specificities for different epitopes on the analyte.

In some embodiments, the labelled reagent is either the analyte itself (e.g., conjugated with a label) or is an analyte analog (e.g., conjugated with a label), e.g., a chemical entity having the identical or substantially and/or effectively the same specific binding characteristics as the analyte. In the latter case, it is preferable that the properties of the analyte analog that influence its solubility or dispersibility in an aqueous liquid sample and its ability to migrate through the moist porous solid phase material are identical or substantially and/or effectively the same as those of the analyte itself. In some embodiments, the labelled analyte or analyte analog migrates through the porous solid phase material into the second zone and binds with the immobilized reagent. An analyte present in the sample competes with the labelled reagent in this binding reaction. Such competition results in a reduction in the amount of labelled reagent binding in the second zone and a consequent decrease in the intensity of the signal observed in the second zone in comparison with the signal that is observed in the absence of analyte in the sample.

In some embodiments, the lateral flow test strip (e.g., the carrier material) comprises nitrocellulose. This has considerable advantage over some other lateral flow test strip materials, such as paper, because it has a natural ability to bind proteins without requiring prior sensitization. Specific binding reagents, such as immunoglobulins, can be applied directly to nitrocellulose and immobilized thereon. No chemical treatment is required that might interfere with the essential specific binding activity of the reagent. Unused binding sites on the nitrocellulose can thereafter be blocked using simple materials, such as polyvinylalcohol. Moreover, nitrocellulose is readily available in a range of pore sizes and this facilitates the selection of a carrier material to suit particularly requirements such as sample flow rate.

In some embodiments, the technology comprises use of one or more "direct labels" attached to one of the specific binding reagents. In some embodiments, the technology uses a label comprising, e.g., colloidal gold (e.g., a sol or colloidal suspension of gold particles (e.g., gold nanoparticles) in a fluid, usually water or an aqueous buffer) or a dye (e.g., a dye sol). In some embodiments, a label produces an instant analytical result without the need to add further reagents to develop a detectable signal. They are robust and stable and can therefore be used readily in an analytical device that is stored in the dry state. Their release on contact with an aqueous sample can be modulated, for example, by the use of soluble glazes.

In some embodiments, development of the devices described herein involves the selection of technical features that enable a direct labelled specific binding reagent to be used in a carrier-based assay device, e.g. one based on a lateral flow assay test strip format, to give a quick and clear result. Ideally, the result of the assay should be discernable by eye and to facilitate this the technology provides for the direct label to become concentrated in the detection zone. Accordingly, the direct labelled reagent is transportable easily and rapidly by the developing liquid. Furthermore, it is preferable that the whole of the developing sample liquid is directed through a comparatively small detection zone so that the probability of an observable result being obtained is increased.

In some embodiments, the technology comprises use of a directly labelled specific binding reagent on a carrier material comprising nitrocellulose. In some embodiments, the nitrocellulose has a pore size of at least one micron. In some embodiments, the nitrocellulose has a pore size not greater than about 20 microns. In some embodiments, the nitrocellulose has a pore size of 1 to 20 microns (e.g., 1, 2, 3, 4, 5, 6, 7, 8, 9, 10, 11, 12, 13, 14, 15, 16, 17, 18, 19, or 20 microns). In some embodiments, the direct label is a colored latex particle of spherical or near-spherical shape and having a maximum diameter of not greater than about 0.5 micron. In some embodiments, the size range for such particles is from about 0.05 to about 0.5 microns (e.g., 0.05, 0.10, 0.15, 0.20, 0.25, 0.30, 0.35, 0.40, 0.45, or 0.50 microns).

In some embodiments, the porous solid phase material is linked to a porous receiving member to which the liquid sample can be applied and from which the sample can permeate into the porous solid phase material. In some embodiments, the porous solid phase material is contained within a moisture-impermeable casing or housing and the porous receiving member, with which the porous solid phase material is linked, extends out of the housing and acts as a means for permitting a liquid sample to enter the housing and permeate the porous solid phase material. In some embodiments, the housing is provided with means, e.g. appropriately placed apertures, that enable the second zone of the porous solid phase material (carrying the immobilized unlabeled specific binding reagent) to be observable from outside the housing so that the result of the assay can be observed. If desired, the housing may also be provided with further means which enable a further zone of the porous solid phase material to be observed from outside the housing and which further zone incorporates control reagents that enable an indication to be given as to whether the assay procedure has been completed.

In some embodiments, assay devices are provided as kits suitable for use in a hospital, clinic, or home. In some embodiments, kits comprise a plurality (e.g., two) of devices individually wrapped in moisture impervious wrapping and packaged together with appropriate instructions to the user.

In some embodiments, the assay device is a self-test assay device, e.g., for use by a user at home or in a clinic in which the results of a self-test assay are directly read by a user inspecting one or more windows overlying the assay detection zones, e.g., to determine the presence or absence of a detectable signal at one or more (or all) of the detection zones. Each detection zone may be provided with a separate window in a housing to allow a user to inspect the detection zone. Alternatively, a large window may accommodate two or more (or all) of the detection zones. Typically, in such user-read devices, the user will directly inspect the detection zones of the assay device or lateral flow assay test strip (e.g., by visual inspection using the user's eyes). In some embodiments, a user determines a result by reference to a color chart or indicator. Conveniently, in some embodiments, the self-test assay device is provided with instructions or guidance for reading the self-test assay result. For example, the user may be provided with a printed color chart to facilitate interpretation of such directly-read visual tests. In some embodiments, the device interprets the assay results for the user.

In some embodiments, a self-test assay device is used with an assay result reading device, which may be a dedicated reading device or a mobile phone or other portable electronic device (e.g. a tablet computer), preferably provided with a camera, where the self-test assay result is read by measuring the signal intensity, e.g., as generated by a visible label. Accordingly, in some embodiments, the assay result reading device may read and interpret the self-test assay results or may transmit self-test assay results data to a remotely-located device for the self-test assay data to be interpreted. The self-test assay data may be transmitted to the remotely-located device in real time. The self-test data may be transmitted via an internet connection or may be stored on a memory device (such as a "flash" drive or the like) which is physically transported to the remote device, or the self-test assay data may be transmitted by wireless communication means (e.g. Bluetooth, near field communication (NFC), or the like). In some embodiments, a microprocessor may control the operation of the optical reading or other self-test assay reading components and will conveniently be programmed with, or be able to access, relevant assay signal threshold values for each of the analytes, compare the actual self-test assay signal values with the predetermined thresholds, and interpret the self-test assay results so as to determine the outcome of the assay. In some embodiments, the self-test assay results are associated with information identifying the user of the device (e.g., an identification number). In some embodiments, the self-test assay results and/or the information identifying the user of the device are encrypted.

In some embodiments, the assay device comprises a porous sample receiving member (e.g., in fluid communication with a lateral flow assay test strip). In some embodiments, the assay device comprises a hollow elongated casing containing a dry porous nitrocellulose carrier that communicates indirectly with the exterior of the casing via a bibulous sample receiving member that protrudes from the casing. In some embodiments, a porous sample receiving member is made from any bibulous, porous, or fibrous material capable of absorbing liquid rapidly. The porosity of the material can be unidirectional (e.g., with pores or fibers running wholly or predominantly parallel to an axis of the member) or multidirectional (omnidirectional, so that the member has an amorphous sponge-like structure). Porous plastics material, such as polypropylene, polyethylene (preferably of very high molecular weight), polyvinylidene fluoride, ethylene vinylacetate, acrylonitrile, and polytetrafluoro-ethylene can be used. It can be advantageous to pre-treat the member with a surface-active agent during manufacture, e.g., to reduce any inherent hydrophobicity in the member and therefore enhance its ability to take up and deliver a moist sample rapidly and efficiently. Porous sample receiving members can also be made from paper or other cellulosic materials, such as nitrocellulose. Materials that are now used in the nibs of so-called fiber tipped pens are particularly suitable and such materials can be shaped or extruded in a variety of lengths and cross-sections appropriate in the context of the invention. In some embodiments, the material comprising the porous receiving member is chosen such that the porous member can be saturated with aqueous liquid within a matter of seconds. Preferably the material remains robust when moist, and for this reason paper and similar materials are less preferred in any embodiment wherein the porous receiving member protrudes from a housing. The liquid must thereafter permeate freely from the porous sample receiving member into the porous solid phase material.

In some embodiments, the assay device comprises an optional "control zone" (e.g., a lateral flow assay test strip comprises a "control zone"). See, e.g., FIG. 2D. If present, the "control" zone can be designed to convey an unrelated signal to the user that the device has functioned properly. For example, the control zone can be loaded with an antibody (e.g., goat anti-rabbit IgG) that will bind to a labelled antibody from the first zone, e.g., a labeled rabbit IgG, to confirm that the sample has permeated the lateral flow assay test strip. In some embodiments, the first zone comprises an antigen and/or antibody that is unrelated to the analyte and that is specifically captured at the control zone. In some embodiments, the control zone can contain an anhydrous reagent that, when moistened, produces a color change or color formation, e.g., an anhydrous copper sulphate that turns blue when moistened by an aqueous sample. As a further alternative, a control zone could contain immobilized analyte that reacts with excess labelled reagent from the first zone. As the purpose of the control zone is to indicate to the user that the test has been completed, the control zone should be located downstream from the second zone in which the desired test result is recorded. A positive control indicator therefore tells the user that the sample has permeated the required distance through the assay device (e.g., through a lateral flow assay test strip of the assay device).

The label can be any entity the presence of which can be readily detected. In some embodiments, the label is a direct label, e.g., an entity that, in its natural state, is readily visible either to the naked eye or with the aid of an optical filter and/or applied stimulation, e.g., UV light to promote fluorescence. For example, minute colored particles, such as dye sols, metallic sols (e.g. gold), and colored latex particles, are very suitable. Concentration of the label into a small zone or volume gives rise to a readily detectable signal, e.g., a strongly-colored area. This can be evaluated by eye, or by instruments if desired.

In some embodiments, the technology comprises use of an indirect label. Indirect labels, such as enzymes, e.g. alkaline phosphatase and horseradish peroxidase, can be used but these usually require the addition of one or more developing reagents such as substrates before a visible signal can be detected. Such additional reagents can be incorporated in the porous solid phase material or in the sample receiving member, if present, such that they dissolve or disperse in the aqueous liquid sample. Alternatively, the developing reagents can be added to the sample before contact with the porous material or the porous material can be exposed to the developing reagents after the binding reaction has taken place.

Coupling of the label to a specific binding reagent can be by covalent bonding, if desired, or by hydrophobic bonding.

According to the technology, the labelled reagent migrates with the liquid sample as it progresses to the detection zone. In some embodiments, the flow of sample continues beyond the detection zone and sufficient sample is applied to the porous material so that this may occur and that any excess labelled reagent from the first zone that does not participate in any binding reaction in the second zone is flushed away from the detection zone by this continuing flow. If desired, an absorbent "sink" can be provided at the distal end of the carrier material (e.g., at the distal end of the lateral flow assay test strip). The absorbent sink may comprise, for example, Whatman 3 MM chromatography paper and should provide sufficient absorptive capacity to allow any unbound conjugate to wash out of the detection zone. As an alternative to such a sink, it can be sufficient to have a length of porous solid phase material which extends beyond the detection zone.

In some embodiments, the presence or intensity of the signal from the label that becomes bound in the second zone provides a qualitative or quantitative measurement of analyte in the sample. A plurality of detection zones arranged in series on the porous solid phase material, through which the aqueous liquid sample can pass progressively, can also be used to provide a quantitative measurement of the analyte, or can be loaded individually with different specific binding agents to provide a multi-analyte test.

In some embodiments, the immobilized specific binding reagent in the second zone is a highly specific antibody (e.g., a monoclonal antibody). In the embodiment of the technology involving the sandwich reaction, the labelled reagent is also a highly specific antibody (e.g., a monoclonal antibody).

In some embodiments, the carrier material is in the form of a strip (e.g., a lateral flow assay test strip) or sheet to which the reagents are applied in spatially distinct zones and the liquid sample is allowed to permeate through the sheet or strip from one side or end to another.

In some embodiments, an assay device according to the technology incorporates two or more discrete bodies of porous solid phase material, e.g. separate lateral flow assay test strips or sheets, each carrying mobile and immobilized reagents. These discrete bodies can be arranged in parallel, for example, such that a single application of liquid sample to the assay device initiates sample flow in the discrete bodies simultaneously. The separate analytical results that can be determined in this way can be used as control results. If different reagents are used on the different carriers, the simultaneous determination of a plurality of analytes in a single sample can be made. Alternatively, multiple samples can be applied individually to an array of carriers and analyzed simultaneously.

In some embodiments, the material comprising the porous solid phase is nitrocellulose. This has the advantage that the antibody in the second zone can be immobilized firmly without prior chemical treatment. If the porous solid phase material comprises paper, for example, the immobilization of the antibody in the second zone needs to be performed by chemical coupling using, for example, cyanogen bromide (CNBr), carbonyldiimidazole, or tresyl chloride.

Following the application of the antibody to the detection zone, the remainder of the porous solid phase material is treated to block any remaining binding sites elsewhere. Blocking can be achieved by treatment with protein (e.g. bovine serum albumin or milk protein) or with polyvinylalcohol or ethanolamine, or any combination of these agents, for example. The labelled reagent for the first zone can then be dispensed onto the dry carrier and will become mobile in the carrier when in the moist state. Between each of these various process steps (sensitization, application of unlabeled reagent, blocking and application of the labelled reagent), the porous solid phase material is dried.

In some embodiments, the labelled reagent is applied to the carrier as a surface layer rather than being impregnated in the thickness of the carrier, e.g., to assist the free mobility of the labelled reagent when the porous carrier is moistened with the sample. This can minimize interaction between the carrier material and the labelled reagent. In some embodiments, the carrier is pre-treated with a glazing material in the region to which the labelled reagent is to be applied. Glazing can be achieved, for example, by depositing an aqueous sugar or cellulose solution, e.g. of sucrose or lactose, on the carrier at the relevant portion, and drying. The labelled reagent can then be applied to the glazed portion. In some embodiments, the remainder of the carrier material is not be glazed.

In some embodiments, the porous solid phase material is a nitrocellulose sheet having a pore size of at least about 1 micron, e.g., greater than about 5 microns (e.g., about 8-12 microns). In some embodiments, the nitrocellulose sheet has a nominal pore size of up to approximately 12 microns (e.g., 1-12 microns (e.g., 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, or 12.0 microns); 5-12 microns (e.g., 5.0, 5.5, 6.0, 6.5, 7.0, 7.5, 8.0, 8.5, 9.0, 9.5, 10.0, 10.5, 11.0, 11.5, or 12.0 microns); 8-12 microns (e.g., 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, or 12.0 microns); and/or 0.01 to 12 microns (e.g., 0.01, 0.05, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 10.1, 10.2, 10.3, 10.4, 10.5, 10.6, 10.7, 10.8, 10.9, 11.0, 11.1, 11.2, 11.3, 11.4, 11.5, 11.6, 11.7, 11.8, 11.9, or 12.0 microns)).

In some embodiments, the nitrocellulose sheet is "backed", e.g. with a plastic sheet, to increase its handling strength. This can be manufactured easily by forming a thin layer of nitrocellulose on a sheet of backing material. The actual pore size of the nitrocellulose when backed in this manner will tend to be, lower than that of the corresponding unbacked material. In some embodiments, a pre-formed sheet of nitrocellulose can be tightly sandwiched between two supporting sheets of solid material, e.g. plastic sheets.

In some embodiments, the flow rate of an aqueous sample through the porous solid phase material is such that in the untreated material, aqueous liquid migrates at a rate of approximately 1 cm in not more than 2 minutes, but slower flow rates can be used if desired. In some embodiments, the spatial separation between the zones, and the flow rate characteristics of the porous carrier material, are selected to allow adequate reaction times during which the necessary specific binding can occur, and to allow the labelled reagent in the first zone to dissolve or disperse in the liquid sample and migrate through the carrier. Further control over these parameters can be achieved by the incorporation of viscosity modifiers (e.g. sugars and modified celluloses) in the sample to slow down the reagent migration.

In some embodiments, the immobilized reagent in the second zone is impregnated throughout the thickness of the carrier in the second zone (e.g., throughout the thickness of the sheet or strip if the carrier is in this form). Such impregnation can enhance the extent to which the immobilized reagent can capture any analyte present in the migrating sample.

The reagents can be applied to the carrier material in a variety of ways. Various "printing" techniques have previously been proposed for application of liquid reagents to carriers, e.g. micro-syringes, pens using metered pumps, direct printing and ink-jet printing, and any of these techniques can be used in the present context. To facilitate manufacture, the carrier (e.g., sheet) can be treated with the reagents and then subdivided into smaller portions (e.g. small narrow strips each embodying the required reagent-containing zones) to provide a plurality of identical carrier units.

Accordingly, embodiments of the technology provide a lateral flow assay test strip. At one end of the lateral flow assay test strip is the sample site to which the sample is to be applied. This sample site comprises a sample pad to which the sample is transferred. Incorporated in the sample site or sample pad, or downstream from the sample site is a labeled antigen, for which the sample is being tested. In some embodiments of the assay technology provided herein, the assay device comprises a labeled pathogen antigen. In some embodiments, the labeled pathogen antigen is labeled pathogen or a labeled pathogen component or part. In some embodiments, the assay device comprises a labeled portion, fragment, epitope, and/or domain of any of the foregoing.

In some embodiments, metal sol particles are prepared by coupling the analyte directly to a gold particle. Additionally, in some embodiments, the labeled component may be prepared by coupling the analyte to the particle using a biotin/avidin linkage. In this latter regard, the substance may be biotinylated and the metal containing particle coated with an avidin compound. The biotin on the analyte may then be reacted with the avidin compound on the particle to couple the substance and the particle together. In another alternative embodiment, the labeled component may be prepared by coupling the analyte to a carrier such as bovine serum albumin (BSA), keyhole lymphocyananin (KLH), or ovalbumin and using this to bind to the metal particles.

In some embodiments, the metal sol particles are prepared by methodologies which are well known. For instance, the preparation of gold sol particles is disclosed in an article by G. Frens, Nature, 241, 20-22 (1973). Additionally, the metal sol particles may be metal or metal compounds or polymer nuclei coated with metals or metal compounds, as described in U.S. Pat No. 4,313,734. Other methods well known in the art may be used to attach the analyte to gold particles. The methods include but are not limited to covalent coupling and hydrophobic bonding. The metal sol particles may be made of platinum, gold, silver, selenium, or copper or any number of metal compounds which exhibit characteristic colors.

In some embodiments, the analyte is not attached to a metal sol particle but is instead attached to dyed or fluorescent labeled microparticles such as latex, polystyrene, dextran, silica, polycarbonate, methylmethacrylates, or carbon. The metal sol particles, dyed particles, or fluorescent labeled microparticles should be visible to the naked eye or able to be read with an appropriate instrument (spectrophotometer, fluorescent reader, and/or an assay result reading device, etc., which may be a dedicated reading device or a mobile phone or other portable electronic device (e.g. a tablet computer), preferably provided with a camera, where the self-test assay result is read by measuring the signal intensity, e.g., as generated by a visible label). Various embodiments provide a number of ways in which the gold labeled antigens are deposited on the lateral flow assay test strip. For example, in some embodiments, the gold labeled antigens/antibodies are deposited and dried on a rectangular or square absorbent pad and the absorbent pad is positioned downstream from where the sample is applied on the lateral flow assay test strip. In some embodiments, the analytes are attached to microspheres. This has the effect of increasing the number of reactive sites (epitopes) in a given area. Analytes may be attached to these alternate solid phases by various methodologies. In some embodiments, hydrophobic or electrostatic domains in the protein are used for passive coating. A suspension of the spheres is mixed after sonication with the antigens/antibodies in water or in a phosphate buffer solution, after which they are incubated at room temperature for 10-75 minutes. The mixture is then centrifuged and the pellets containing the antigen/antibody-linked microspheres are suspended in a buffer containing 1-5% wt/volume bovine serum albumin (BSA) for 1 hour at room temperature. The BSA blocks any unreacted surfaces of the microspheres. After one more centrifugation, the spheres are resuspended in buffer (TBS with 5% BSA) and stored at 4 degrees C before using.

In some embodiments, the solid phase particles comprise water dispersable particles, such as polystyrene latex particles disclosed in U.S. Pat. No. 3,088,875, incorporated herein by reference. Such solid phase materials simply consist of suspensions of small, water-insoluble particles to which antigens/antibodies are able to bind. Suitable solid phase particles are also disclosed, for example, in U.S. Pat. Nos. 4,184,849; 4,486,530; and 4,636,479.

In some embodiments, analytes are attached to fluorescent microspheres or fluorescent microparticles. Characteristically, fluorescent microspheres incorporate fluorescent dyes in the solid outer matrix or in the internal volume of the microsphere. The fluorescent spheres are typically detected by a fluorescent reader that excites molecules at one wavelength and detects the emission of fluorescent waves at another wavelength. For example, Nile Red particles excite at 526 nm at emit at 574 nm, the Far Red excites at 680 nm and emits at 720 nm, and the Blue excites at 365 nm and emits at 430 nm. In a lateral flow assay format, in some embodiments, detection of fluorescent microparticles involves the use of a reflectance reader with an appropriate excitation source (e.g., HeNe, Argon, tungsten, or diode laser) and an appropriate emission filter for detection. Use of diode lasers allows for use of detection systems that use low cost lasers with detection above 600 nm. Most background fluorescence is from molecules that emit fluorescence below 550 nm.

In some embodiments, fluorescent microspheres comprise surface functional groups such as carboxylate, sulfate, or aldehyde groups, making them suitable for covalent coupling of proteins and other amine containing biomolecules. In addition, sulfate, carboxyl and amidine microspheres are hydrophobic particles that will passively absorb almost any protein or lectin. Coating is thus similar as for nonfluorescent microspheres. In some embodiments, a suspension of the fluorescent spheres is mixed after sonication with the antigens/antibody in water or in a phosphate buffered solution, after which they are incubated at room temperature for 10-75 minutes. EDAC (soluble carbodiimide), succinimidyl esters, and isothiocyanates, as well as other crosslinking agents, may be used for covalent coupling of proteins and lectins to the microspheres. After the protein has attached to the surface of the miroparticles, the mixture is centrifuged and the pellets containing the antigen or antibody linked to the fluorescent microparticles are suspended in a buffer containing 1-5% bovine serum albumin for one hour. After one more centrifugation, the spheres are resuspended in buffer (TBS with 5% BSA or other appropriate buffers) and stored at 4 degrees C before use.

In some embodiments, the solid phase particles comprise, for example, particles of latex or of other support materials such as silica, agarose, glass, polyacrylamides, polymethyl methacrylates, carboxylate modified latex and Sepharose. Preferably, the particles vary in size from about 0.2 microns to about 10 microns (e.g., 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0 microns). In some embodiments, particles are coated with a layer of antigens coupled thereto in a manner known per se in the art to present the solid phase component.

Accordingly, embodiments provide that a sample comprising antibodies (e.g., IgG and/or IgM) to a pathogen (e.g., a microbe such as a virus, prokaryote (e.g., bacterium), or eukaryote (e.g., fungus or protozoan parasite)) reacts with the labeled antigen to form an antigen-antibody complex (e.g., a labeled antigen-antibody complex). The antigen-antibody complex begins to migrate along the lateral flow assay test strip. The antigen-antibody complex begins to migrate along the lateral flow assay test strip. In some embodiments, further down the length of the lateral flow assay test strip are three binding sites. A first binding site preferably binds IgM. A second binding site preferably binds IgG. A third binding site is for a control. More specifically, each binding site is in the form of a striped line along the width of the lateral flow assay test strip. Each binding site comprises an antibody. For example, in some embodiments, an anti-human IgM antibody is laid down at the first binding site and an anti-human IgG antibody is laid down at the second site. At the control site there is immobilized an antibody to a control substance (e.g., a labeled antibody or antigen). In some embodiments, the antibodies that bind with IgM and IgG are from affinity purification of immune sera from goats, rabbits, donkeys, sheep, chickens, or other animals. In some embodiments, the antibodies that bind with IgM and IgG are monoclonal antibodies directed against IgM and IgG. In some embodiments, the antibodies used are specific for the heavy chain portion of the IgM and IgG antibodies.

In some embodiments, a sample comprising a pathogen (e.g., a microbe such as a virus, prokaryote (e.g., bacterium), or eukaryote (e.g., fungus or protozoan parasite)) or a pathogen antigen (e.g., antigen and/or component or part of a microbe such as a virus, prokaryote (e.g., bacterium), or eukaryote (e.g., fungus or protozoan parasite)) reacts with an antibody (e.g., a labeled antibody) to form an antigen-antibody complex (e.g., a labeled antigen-antibody complex). The antigen-antibody complex begins to migrate along the lateral flow assay test strip. See, e.g., FIG. 1. In some embodiments, further down the length of the lateral flow assay test strip are two or three binding sites. A first binding site comprises an immobilized antibody specific for the pathogen (e.g., a microbe such as a virus, prokaryote (e.g., bacterium), or eukaryote (e.g., fungus or protozoan parasite)) or a pathogen antigen (e.g., antigen and/or component or part of a microbe such as a virus, prokaryote (e.g., bacterium), or eukaryote (e.g., fungus or protozoan parasite)). At a control site there is immobilized an antibody to a control substance (e.g., a labeled antibody or antigen). Although the disclosure herein refers to certain illustrated embodiments, it is to be understood that these embodiments are presented by way of example and not by way of limitation.

### Examples

### Self-test device comprising a sample transfer component

During the development of embodiments of the technology described herein, embodiments (e.g., 200A and 200B) of a self-test device were designed and tested that collect a quantitatively measured volume of blood for testing and displays whether the collected amount is sufficient during the collection process. In particular, the assay device comprises a sample collection port 213 comprising a pedestal 201 for sample collection (FIG. 2A and FIG. 2B) fluidly connected to a blood transfer part 202 (FIG. 2A and 2B) comprising a blood sample capillary tube and compressible chamber 206 (FIG. 2C). The sample collection port 213 (e.g., comprising pedestal 201) is adapted to collect a blood sample directly from a human finger, e.g., by allowing drops of blood to contact and/or enter the sample collection port as they are dropped from a finger (e.g., a lanced finger).

As shown in FIG. 2A and 2B, in some embodiments, the self-test device 200A and/or 200B comprises a top panel 250 and a bottom panel 260. In some embodiments, the top panel comprises a signal window 209, a buffer well 210, a test results viewing window 211, a sample collection port 213, and a window 214 for a user of the self-test device to access (e.g., contact and compress) a compressible chamber (see FIG. 2C, 206). When assembled (e.g., by coupling the top panel 250 and bottom panel 260), the lateral flow assay test strip 212, the pedestal 201, and the blood transfer part 202 are held in place between the assembled top panel 250 and bottom panel 260. In some embodiments, the bottom panel 260 comprises raised features that hold the lateral flow assay test strip 212, the pedestal 201, and the blood transfer part 202 in place. The sample collection port 213 comprises the pedestal 201 upon which a sample is provided. The pedestal 201 is in fluid communication with the outside of the device and thus, when a patient provides a blood sample from a finger, the pedestal is in fluid communication with the patient finger (e.g., with the patient blood). The pedestal 201 is in fluid communication with the blood transfer part 202 (e.g., the large end 205 of the blood transfer part 202). The small end 203 of the blood transfer part 202 is in fluid communication with the buffer well 210. The buffer well 210 is in fluid communication with the lateral flow assay test strip 212.

While the arrangement of the features provided in the top panel 250, bottom panel 260, and held between the top panel 250 and bottom panel 260 (e.g., lateral flow assay test strip 212, the pedestal 201, and the blood transfer part 202) may vary (e.g., as shown in embodiments 200A and 200B), the technology provides that a blood sample is provided through a sample collection port 213 to a pedestal, the blood sample is delivered by the pedestal 213 to the blood transfer part 202, and by operation of a compressible chamber (see FIG. 2C, 206), a metered sample of the blood is provided to the buffer well 210 to provide a buffered blood sample. Provision of buffer to the buffer well 210 initiates assay of the buffered blood sample on the lateral flow assay test strip 212. Accordingly, the technology is not limited to the embodiments shown as embodiment 200A and 200B provided that the sample is provided as described and flows through the components as described.

For example, in some embodiments, the sample collection port 213 comprises a "pedestal" 201 comprising grooves that direct a blood sample to the blood transfer part 202. The pedestal 201 comprises a main central groove and one or more transverse grooves that are fluidly connected to the main central groove. The top surface of the pedestal 201 is concave such that a blood sample pools within the concave surface of the pedestal 201. The pedestal 201 comprises a ramp component that is fluidly connected to the blood transfer part 202. The ramp component comprises at least a portion of the main central groove. Blood that pools within the pedestal 201 is pulled by gravity to move within the main central groove down the ramp component to the blood transfer part. The transverse grooves help to transport blood pooled within the concave surface of the pedestal 201 to the main central groove for transport to the blood transfer part 202. After transfer of the blood sample to the blood transfer part 202, the blood sample capillary tube fills with blood.

The pedestal is in fluid communication with a blood sample capillary tube. Accordingly, the blood sample capillary tube fills with a blood sample provided to the assay device (e.g., provided to the pedestal 201). The blood sample capillary tube comprises a small end 203 (FIG. 2C) fluidly connected to a center portion 204 (FIG. 2C) and a large end 205 (FIG. 2C) fluidly connected to the center portion 204. In the small end 203 of the blood sample capillary tube, capillary forces draw blood into the blood sample capillary tube from the sample collection port comprising a pedestal 201 and the blood moves into the center portion 204 and/or to the large end 205 of the blood sample capillary tube. Capillary forces in the large end 205 of the blood sample capillary tube are negligible and/or nonexistent.

The small end 203 of the blood sample capillary tube is fluidly connected to the sample collection port comprising a pedestal 201 and the large end 205 is fluidly connected to a buffer well 210, which is, in turn, fluidly connected to a lateral flow assay test strip 212 (e.g., a sample pad of a lateral flow assay test strip). The center portion 204 is fluidly connected to a compressible chamber 206 (FIG. 2C) (e.g., a compressible chamber comprising air). The blood sample capillary tube (e.g., the small end 203 of the blood sample capillary tube) is fluidly connected to the sample collection port comprising a pedestal 201 such that blood contacting the sample collection port comprising a pedestal 201 is moved into the blood sample capillary tube by capillary action. Thus, a blood sample provided to the sample collection port comprising a pedestal 201 (e.g., by lancing a finger and touching the resulting blood sample to the sample collection port comprising a pedestal 201) moves from the sample collection port comprising a pedestal 201 to the blood sample capillary tube, thus filling at least a portion of the blood sample capillary tube with blood. The assay device comprises a signal window 209 through which the blood sample capillary tube is visible. When a user observes blood in the portion of the blood sample capillary tube that is visible through the signal window 209, a sufficient amount of blood has been provided to the assay device. After confirming (e.g., by visual inspection of the signal window 209) that a sufficient amount of blood has been collected in the blood sample capillary tube, blood sample is mixed with a buffer by providing a buffer into buffer well 210, which provides a buffered blood sample. The buffered blood sample is thus provided to a lateral flow assay test strip 212 by capillary action.

In particular, the collected blood sample (e.g., in the blood sample capillary tube) is transferred to a buffer well 210 by compressing the compressible chamber 206. In some embodiments, the device comprises a blood transfer button operably connected to the compressible chamber 206. The compressible chamber 206 comprises air. When the compressible chamber 206 is compressed, air in the chamber is moved into the blood sample capillary tube and blood in the blood sample capillary tube moves toward and out of the large end 205 of the blood sample capillary tube. When pressure is applied to the blood in the center portion 204 of the blood sample capillary tube (e.g., by compressing the compressible chamber 206 in fluid communication with the center portion 204 of the blood sample capillary tube), the large end 205 of the blood sample capillary tube provides lower resistance to blood flow than the small end 203 of the blood sample capillary tube. In particular, the head pressure at the small end 203 of the blood sample capillary tube is larger than the head pressure at the large end 205 of the blood sample capillary tube because the small end 203 of the blood sample capillary tube has a smaller diameter and/or area (FIG. 2C, 207) than the inner diameter and/or area of the large end (FIG. 2C, 208) of the blood sample capillary tube. Furthermore, capillary pressure in the small end 203 of the blood sample capillary provides a driving force for fluid transport of blood toward the large end 205 of the blood sample capillary tube. Thus, a metered portion of the blood flows toward the large end 205 of the blood sample capillary tube. In some embodiments, the inner diameter 207 of the small end 203 of the blood sample capillary tube is approximately 0.5 mm (e.g., 0.40, 0.41, 0.42, 0.43, 0.44, 0.45, 0.46, 0.47, 0.48, 0.49, 0.50, 0.51, 0.52, 0.53, 0.54, 0.55, 0.56, 0.57, 0.58, 0.59, or 0.60 mm) and the inner diameter 208 of the large end 205 of the blood sample capillary tube is approximately 1.1 mm (e.g., 1.00, 1.01, 1.02, 1.03, 1.04, 1.05, 1.06, 1.07, 1.08, 1.09, 1.10, 1.11, 1.12, 1.13, 1.14, 1.15, 1.16, 1.17, 1.18, 1.19, or 1.20 mm). In some embodiments, the length of the blood sample capillary tube (e.g., from the small end to the large end) is approximately 42.8 mm (e.g., 42.0, 42.1, 42.2, 42.3, 42.4, 42.5, 42.6, 42.7, 42.8, 42.9, 43.0, 43.1, 43.2, 43.3, 43.4, 43.5, 43.6, 43.7, 43.8, 43.9, or 44.0 mm).

Compressing the compressible chamber 206 moves a metered portion (e.g., comprising approximately 1, 2, 3, 4, or 5 drops (e.g., approximately 50 to 250 microliters (e.g., 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, or 250 microliters))) of the blood sample to a buffer well 210. In some embodiments, the device comprises a transfer button (not shown) and pressing the transfer button compresses the compressible chamber 206. The compressible chamber 206 in turn compresses the blood sample capillary tube to drive a metered portion (e.g., comprising approximately 1, 2, 3, 4, or 5 drops (e.g., approximately 50 to 250 microliters (e.g., 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, or 250 microliters))) of the blood sample to a buffer well 210 of the assay device. In some embodiments, compressing the compressible chamber 206 moves air that drives a metered portion (e.g., comprising approximately 1, 2, 3, 4, or 5 drops (e.g., approximately 50 to 250 microliters (e.g., 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, or 250 microliters))) of the blood sample to a buffer well 210 of the assay device.

Next, buffer (e.g., approximately 1, 2, 3, 4, or 5 drops (e.g., approximately 50 to 250 microliters (e.g., 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, or 250 microliters))) is provided into a buffer well 210 of the assay device. The buffer mixes with the blood sample in the buffer well 210 to provide a buffered blood sample. The buffered blood sample contacts the lateral flow assay test strip 212 to initiate the lateral flow assay on the lateral flow assay test strip 212. A test result is read after a time interval passes that is adequate for development of the lateral flow assay test strip. The appropriate time interval may vary depending on the analyte that is tested (e.g., detected) by the lateral flow assay and/or by the particular design of the assay device. In some embodiments, the test result is read after a time of 5 to 40 minutes (e.g., 5, 10, 15, 20, 25, 30, 35, or 40 minutes) has passed since initiating the lateral flow assay. In some preferred embodiments, the test result is read after 15 minutes and before 20 minutes of initiating the lateral flow assay (e.g., within 15-20 minutes (e.g., after 15.0, 15.5, 16.0, 16.5, 17.0, 17.5, 18.0, 18.5, 19.0, 19.5, or 20.0 minutes) of initiating the lateral flow assay (e.g., within 15-20 minutes (e.g., after 15.0, 15.5, 16.0, 16.5, 17.0, 17.5, 18.0, 18.5, 19.0, 19.5, or 20.0 minutes) of providing the buffer into the buffer well)). A user observes the formation of a color at a test results line (e.g., for the presence of IgM ("M") and/or IgG ("G")) and/or at a control line. See, e.g., FIG. 2D.

In some embodiments, a user starts a timer immediately after initiating the lateral flow assay and reads a test result (e.g., through a test results viewing window 211) after the timer indicates the end of the time interval.

Thus, embodiments of the technology relate to a method comprising obtaining a blood sample from a patient, observing blood through a signal window, and compressing a compressible chamber (e.g., by pressing a blood transfer component (e.g., button)) in fluid communication with a sample collection port and blood sample capillary tube to advance the blood sample from a blood sample capillary tube to a buffer well. In some embodiments, a user adds buffer to the sample (e.g., by providing buffer into a buffer well of the assay device). The buffer mixes with the blood sample and the buffered blood sample is then advanced into the lateral flow assay test strip using wicking action provided by the lateral flow assay test strip. Accordingly, providing a buffer (e.g., into a buffer well) initiates a lateral flow assay on a lateral flow assay test strip. Thus, the technology provided herein simplifies the operation steps and is convenient for users to operate.

In particular, in some embodiments, the technology provides quantitative collection due to the capillary principle filling the blood sample capillary tube. The two ends of the blood sample capillary tube are large and small and air is blown into the middle. Upon applying air pressure to the portion of the sample in the center of the blood sample capillary tube, liquid will only flow toward and out from the large end, which provides sample transfer of a metered amount of sample to the buffer well and subsequently to the lateral flow assay test strip. Thus, in some embodiments, the technology provides that there is no need to use a pipette to transfer a quantified (e.g., metered) amount of a blood sample; blood collection is quantified and verified by observation of blood in the signal window (e.g., signaling that blood collection volume is sufficient). Furthermore, parts pressed by users are elastic materials that minimize and/or eliminate the chance of device misoperation. Thus, the technology finds use to transfer quantified specimens (e.g., blood samples) to a reagent strip. During use, a user places the blood on a sample collection port and transfers a metered blood sample to the lateral flow assay test strip using a blood transfer component. See, e.g., FIG. 2A, FIG. 2B, and FIG. 2C.

In some embodiments, the self-test device finds use in a clinic where a tester and/or a user of the self-test device conducts an assay for an analyte, e.g., to test a sample for infection with a pathogen. In some embodiments, the user of the device provides a sample and the user interprets the test results. In some embodiments, the user provides a sample and a health care provider interprets the test results. In some embodiments, a health care provider obtains a sample, the health care provider provides the sample to the self-test device, and the health care provider interprets the test results. Accordingly, in some embodiments, the technology provides a device for consumers (e.g., lay users) to test themselves for infection with a pathogen. In some embodiments, the technology provides a device for a health care provider to test a patient for infection with a pathogen. In some embodiments of the device, blood is collected into a container (e.g., a fixed amount of blood is aspirated by observing a fill line on a pipette, capillary, or specimen dropper) and then transferred to a reagent strip and in some embodiments a blood sample is provided directly to an assay device.

### Serological lateral flow assay

Another exemplary lateral flow assay for detecting antibodies specific for SARS-CoV-2 is provided herein. The assay device comprises housing, a plastic backing, a nitrocellulose membrane, a sample pad, a label pad, and an absorbent bad. See, e.g., FIG. 1. The label pad comprises a recombinant SARS-CoV-2 antigen comprising a label (e.g., a gold or latex colloid). The label pad comprises a rabbit antibody (e.g., IgG) comprising a label (e.g., a gold or latex colloid) for a control reaction. The nitrocellulose membrane comprises a detection region comprising two test lines and a control line. In some embodiments, a first test strip comprises a first test line and a second test strip comprises a second test line. In some embodiments, one test strip comprises the first test line and the second test line. In embodiments comprising a first test strip and a second test strip, the first test strip and/or the second test strip can comprise a control line. The first test line comprises an immobilized mouse anti-human IgG monoclonal antibody and the second test line comprises an immobilized mouse anti-human IgM monoclonal antibody. The control line comprises a goat anti-rabbit IgG monoclonal antibody.

To test for the presence of IgG and/or IgM antibodies to SARS-CoV-2, users obtain a sample of serum, plasma, whole blood, or capillary blood. The sample is applied to the sample pad. Next, two drops of a buffer are applied to the sample pad to start the test. A visible line at the "G" test line indicates the presence of anti-SARS-CoV-2 IgG in the sample. A visible line at the "M" test line indicates the presence of anti-SARS-CoV-2 IgM in the sample. A visible line at the "C" control line indicates that the test performed correctly. A lack of a visible line at the "C" control line indicates an invalid test. See, e.g., FIG. 2D. Various modifications and variations of the described compositions, methods, and uses of the technology will be apparent to those skilled in the art. Although the technology has been described in connection with specific exemplary embodiments, it should be understood that the invention as claimed should not be unduly limited to such specific embodiments.

## Claims

1. An assay device (200A, 200B) comprising:
a lateral flow assay test strip (212);
a capillary tube comprising a large end (205), a center portion (204), and a small end (203);
a sample collection port in fluid communication with the small end of the capillary tube;
a buffer well (210) in fluid communication with the large end (205) of the capillary tube and with the lateral flow assay test strip (212);
a depressible chamber (206) in fluid communication with the center portion (204) of the capillary tube,
wherein the depressible chamber (206) is adapted to move a sample in the capillary tube into the buffer well (210).

2. The assay device of claim 1, further comprising
a housing comprising a signal window (209) through which is visible at least a portion of the capillary tube;
a transfer button coupled to the compressible chamber (206);
a buffer well (210) in fluid communication with the buffer well; and
a test results viewing window (211) through which is visible at least a portion of the lateral flow test strip (212).

3. The assay device of claim 1, wherein depressing the depressible chamber (206) moves a metered sample in the capillary tube into the buffer well (210) .

4. The assay device of claim 1, wherein the depressible chamber (206) in an expanded state comprises air and the depressible chamber (206) in a depressed state displaces air into the capillary tube to move the sample to the buffer well (210).

5. The assay device of claim 2, wherein providing a buffer into the buffer well (210) provides a buffer in the buffer well (210), said buffer in the buffer well (210) mixes with the sample to provide a buffered sample, and said buffered sample contacts the lateral flow assay test strip (212) to initiate a lateral flow assay.

6. The assay device of claim 1, wherein the lateral flow assay test strip comprises a labeled recombinant antigen; and an anti-human antibody.

7. The assay device of claim 6, wherein the anti-human antibody is immobilized on the lateral flow test strip (212).

8. The assay device of claim 1, wherein the lateral flow assay test strip (212) comprises a first antibody and a second antibody.

9. The assay device of claim 8, wherein the first antibody is immobilized on the lateral flow test strip (212) and the second antibody comprises a label.

## Patentansprüche

1. Eine Assay-Vorrichtung (200A, 200B), die Folgendes umfasst:
einen Lateral-Flow-Assay-Teststreifen (212);
ein Kapillarrohr, das ein großes Ende (205), einen mittleren Abschnitt (204) und ein kleines Ende (203) umfasst;
einen Probenerfassungsport in Fluidkommunikation mit dem kleineren Ende des Kapillarrohrs;
eine Puffervertiefung (210) in Fluidkommunikation mit dem großen Ende (205) des Kapillarrohrs und mit dem Lateral-Flow-Assay-Teststreifen (212);
eine drückbare Kammer (206) in Fluidkommunikation mit dem mittleren Abschnitt (204) des Kapillarrohrs,
wobei die drückbare Kammer (206) angepasst ist, um eine Probe in dem Kapillarrohr in die Puffervertiefung (210) zu bewegen.

2. Die Assay-Vorrichtung nach Anspruch 1, die ferner Folgendes umfasst
ein Gehäuse, das ein Signalfenster (209) umfasst, durch das mindestens ein Abschnitt des Kapillarrohrs sichtbar ist;
einen Transferknopf, der mit der drückbaren Kammer(206) gekoppelt ist;
eine Puffervertiefung (210) in Fluidkommunikation mit der Puffervertiefung; und
ein Testresultatsichtfenster (211), durch das mindestens ein Abschnitt des Lateral-Flow-Teststreifens (212) sichtbar ist.

3. Die Assay-Vorrichtung nach Anspruch 1, wobei sich durch Drücken der drückbaren Kammer (206) eine dosierte Probe in dem Kapillarrohr in die (210) Puffervertiefung bewegt.

4. Die Assay-Vorrichtung nach Anspruch 1, wobei die drückbare Kammer (206) in einem expandierten Zustand Luft umfasst und die drückbare Kammer (206) in einem gedrückten Zustand Luft in das Kapillarrohr verdrängt, um die Probe zur Puffervertiefung (210) zu bewegen.

5. Die Assay-Vorrichtung nach Anspruch 2, wobei das Bereitstellen eines Puffers in die Puffervertiefung (210) einen Puffer in die Puffervertiefung (210) bereitstellt, wobei sich der genannte Puffer in der Puffervertiefung (210) mit der Probe vermischt, um eine gepufferte Probe bereitzustellen, und die genannte gepufferte Probe den Lateral-Flow-Assay-Teststreifen (212) berührt, um ein Lateral-Flow-Assay zu initiieren.

6. Die Assay-Vorrichtung nach Anspruch 1, wobei der Lateral-Flow-Assay-Teststreifen Folgendes umfasst: ein markiertes rekombinantes Antigen; und einen Anti-Human-Antikörper.

7. Die Assay-Vorrichtung nach Anspruch 6, wobei der Anti-Human-Antikörper auf dem Lateral-Flow-Teststreifen (212) immobilisiert ist.

8. Die Assay-Vorrichtung nach Anspruch 1, wobei der Lateral-Flow-Assay-Teststreifen (212) einen ersten Antikörper und einen zweiten Antikörper umfasst.

9. Die Assay-Vorrichtung nach Anspruch 8, wobei der erste Antikörper auf dem Lateral-Flow-Teststreifen (212) immobilisiert ist und der zweite Antikörper einen Marker umfasst.

## Revendications

1. Dispositif d'analyse (200A, 200B) comprenant:
une bandelette de test de dosage à écoulement latéral (212) ;
un tube capillaire comprenant une grande extrémité (205), une portion centrale (204), et une petite extrémité (203);
un port de collecte d'échantillon en communication fluidique avec la petite extrémité du tube capillaire;
un puits à tampon (210) en communication fluidique avec la grande extrémité (205) du tube capillaire et avec la bandelette de test de dosage à écoulement latéral (212);
une chambre apte à être enfoncée (206) en communication fluidique avec la portion centrale (204) du tube capillaire,
dans lequel la chambre apte à être enfoncée (206) est conçue pour amener un échantillon dans le tube capillaire jusque dans le puits à tampon (210).

2. Dispositif d'analyse de la revendication 1, comprenant en outre
un logement comprenant une fenêtre de signaux (209) à travers laquelle au moins une portion du tube capillaire est visible;
un bouton de transfert couplé à la chambre compressible (206);
un puits à tampon (210) en communication fluidique avec le puits à tampon; et
une fenêtre de visualisation des résultats de test (211) à travers laquelle au moins une portion de la bandelette de test à écoulement latéral (212) est visible.

3. Dispositif d'analyse de la revendication 1, dans lequel le fait d'appuyer sur la chambre apte à être enfoncée (206) amène un échantillon dosé dans le tube capillaire jusque dans le puits à tampon (210).

4. Dispositif d'analyse de la revendication 1, dans lequel la chambre apte à être enfoncée (206), dans un état expansé, comprend de l'air et la chambre apte à être enfoncée (206), dans un état enfoncé, déplace l'air jusque dans le tube capillaire afin d'amener l'échantillon vers le puits à tampon (210).

5. Dispositif d'analyse de la revendication 2, dans lequel le fait de mettre à disposition un tampon dans le puits à tampon (210) fournit un tampon dans le puits à tampon (210), ledit tampon dans le puits à tampon (210) se mélange avec l'échantillon afin de procurer un échantillon tamponné, et ledit échantillon tamponné entre en contact avec la bandelette de test de dosage à écoulement latéral (212) afin d'amorcer un dosage à écoulement latéral.

6. Dispositif d'analyse de la revendication 1, dans lequel la bandelette de test de dosage à écoulement latéral comprend un antigène recombinant marqué; et un anticorps anti-humain.

7. Dispositif d'analyse de la revendication 6, dans lequel l'anticorps anti-humain est immobilisé sur la bandelette de test à écoulement latéral (212).

8. Dispositif d'analyse de la revendication 1, dans lequel la bandelette de test de dosage à écoulement latéral (212) comprend un premier anticorps et un deuxième anticorps.

9. Dispositif d'analyse de la revendication 8, dans lequel le premier anticorps est immobilisé sur la bandelette de test à écoulement latéral (212) et le deuxième anticorps comprend un marquage.
